# EUROPÄISCHE PATENTSCHRIFT

(11) **EP 1 244 942 B1**
(45) Veröffentlichungstag und Bekanntmachung des Hinweises auf die Patenterteilung: **01.10.2008**
(21) Anmeldenummer: 00972909.6
(22) Anmeldetag: 14.11.2000
(51) Int. Cl.: G03G 9/097, C09D 5/03, H01G 7/02, B01D 57/00, C07C 211/63, C01B 33/44, C01B 39/02

(54) **VERWENDUNG VON SALZARTIGEN STRUKTURSILIKATEN ALS LADUNGSSTEUERMITTEL**
USE OF SALT-LIKE STRUCTURAL SILICATES AS CHARGE CONTROLLING AGENTS
UTILISATION DE SILICATES STRUCTURAUX DU TYPE SEL COMME AGENTS DE REGULATION DE CHARGE

(30) Priorität: 27.11.1999 DE 19957245
(43) Veröffentlichungstag der Anmeldung: 02.10.2002
(73) Patentinhaber: Clariant Produkte (Deutschland) GmbH, 65929 Frankfurt am Main (DE)
(72) Erfinder: MICHEL, Eduard, 60529 Frankfurt am Main (DE); BAUR, Rüdiger, 65817 Eppstein-Niederjosbach (DE); MACHOLDT, Hans-Tobias, 64297 Darmstadt-Eberstadt (DE)
(86) Internationale Anmeldenummer: PCT/EP2000/011217
(87) Internationale Veröffentlichungsnummer: WO 2001/040878

(56) Entgegenhaltungen:
- DE-A- 3 120 542
- US-A- 4 808 849
- US-A- 5 015 676
- US-A- 5 556 618
- PATENT ABSTRACTS OF JAPAN vol. 1996, no. 05, 31. Mai 1996 (1996-05-31) & JP 08 006295 A (RICOH), 12. Januar 1996 (1996-01-12)
- PATENT ABSTRACTS OF JAPAN vol. 014, no. 529, 20. November 1990 (1990-11-20) & JP 02 221964 A (CANON), 4. September 1990 (1990-09-04)
- DATABASE WPI Section Ch, Week 199041 Derwent Publications Ltd., London, GB; Class E05, AN 1990-308725 XP002159326 & JP 02 217867 A (FUJI XEROX), 30. August 1990 (1990-08-30)
- DATABASE WPI Section Ch, Week 199538 Derwent Publications Ltd., London, GB; Class A12, AN 1995-285780 XP002159327 & JP 07 181719 A (KAO), 21. Juli 1995 (1995-07-21)
- PATENT ABSTRACTS OF JAPAN vol. 009, no. 203, 21. August 1985 (1985-08-21) & JP 60 066262 A (FUJI SHASHIN)
- DATABASE WPI Section Ch, Week 199105 Derwent Publications Ltd., London, GB; Class A12, AN 1991-031921 XP002159328 & JP 02 299878 A (CANON), 12. Dezember 1990 (1990-12-12)

## Beschreibung

Die vorliegende Erfindung liegt auf dem Gebiet der Ladungssteuermittel im Sinne einer Komponente, die das elektrostatische Aufladungsverhalten in einer Matrix selektiv beeinflußt.

Bei elektrophotographischen Aufzeichnungsverfahren wird auf einem Photoleiter ein "latentes Ladungsbild" erzeugt. Dieses "latente Ladungsbild" wird durch Aufbringen eines elektrostatisch geladenen Toners entwickelt, der dann beispielsweise auf Papier, Textilien, Folien oder Kunststoff übertragen und beispielsweise mittels Druck, Strahlung, Hitze oder Lösungsmitteleinwirkung fixiert wird. Typische Toner sind Ein-oder Zweikomponentenpulvertoner (auch Ein- oder Zweikomponentenentwickler genannt), darüber hinaus sind noch Spezialtoner, wie z.B. Magnettoner, Flüssigtoner oder Polymerisationstoner im Einsatz. Unter Polymerisationstonem sind solche Toner zu verstehen, die z. B. durch Suspensionspolymerisation (Kondensation) oder Emulsionspolymerisation entstehen und zu verbesserten Teilcheneigenschaften des Toners führen. Weiterhin sind auch solche Toner gemeint, die grundsätzlich in nichtwäßrigen Dispersionen erzeugt werden.

Ein Maß für die Tonerqualität ist seine spezifische Aufladung q/m (Ladung pro Masseeinheit). Neben Vorzeichen und Höhe der elektrostatischen Aufladung ist das schnelle Erreichen der gewünschten Ladungshöhe, die Konstanz dieser Ladung über einen längeren Aktivierzeitraum sowie die Unempfindlichkeit des Toners gegen Klimaeinflüsse, wie Temperatur und Luftfeuchtigkeit ein wichtiges Qualitätskriterium. Sowohl positiv als auch negativ aufladbare Toner finden Verwendung in Kopierern und Laserdruckern in Abhängigkeit vom Verfahrens- und Gerätetyp.

Um elektrophotographische Toner oder Entwickler mit entweder positiver oder negativer Aufladung zu erhalten, werden häufig Ladungssteuermittel zugesetzt. Da Tonerbindemittel oftmals eine starke Abhängigkeit der Aufladung von der Aktivierzeit aufweisen, ist es Aufgabe eines Ladungssteuermittels, zum einen Vorzeichen und Höhe der Toneraufladung einzustellen und zum anderen der Aufladungsdrift des Tonerbindemittels entgegenzuwirken und für Konstanz der Toneraufladung zu sorgen. Darüber hinaus ist für die Praxis wichtig, daß die Ladungssteuermittel eine ausreichende Thermostabilität und eine gute Dispergierbarkeit besitzen. Typische Einarbeitungstemperaturen für Ladungssteuermittel in die Tonerharze liegen bei Verwendung von Knetern oder Extrudern zwischen 100°C und 200°C. Dementsprechend ist eine Thermostabilität von 200°C von großem Vorteil. Wichtig ist auch, daß die Thermostabilität über einen längeren Zeitraum (ca. 30 Minuten) und in verschiedenen Bindemittelsystemen gewährleistet ist.

Für eine gute Dispergierbarkeit ist es von Vorteil, wenn das Ladungssteuermittel keine wachsartigen Eigenschaften, keine Klebrigkeit und einen Schmelz- oder Erweichungspunkt von > 150°C, besser > 200°C, aufweist. Klebrigkeit führt häufig zu Problemen beim Zudosieren in die Tonerformulierung, und niedrige Schmelz- oder Erweichungspunkte können dazu führen, daß beim Eindispergieren keine homogene Verteilung erreicht wird, da sich das Material tröpfchenförmig im Trägermaterial zusammenschließt.

Typische Tonerbindemittel sind Polymerisations-, Polyadditions- und Polykondensationsharze wie Styrol-, Styrolacrylat-, Styrolbutadien-, Acrylat-, Polyester-, Phenol-Epoxidharze, sowie Cycloolefincopolymere, einzeln oder in Kombination, die noch weitere Inhaltsstoffe, z.B. Farbmittel, wie Farbstoffe und Pigmente, Wachse oder Fließhilfsmittel, enthalten können oder im Nachhinein zugesetzt bekommen können, wie hochdisperse Kieselsäuren.

Ladungssteuermittel können auch zur Verbesserung der elektrostatischen Aufladung von Pulvern und Lacken, insbesondere in triboelektrisch oder elektrokinetisch versprühten Pulverlacken, wie sie zur Oberflächenbeschichtung von Gegenständen aus beispielsweise Metall, Holz, Kunststoff, Glas, Keramik, Beton, Textilmaterial, Papier oder Kautschuk zur Anwendung kommen, eingesetzt werden. Der Pulverlack oder das Pulver erhält seine elektrostatische Aufladung im allgemeinen nach einem der beiden folgenden Verfahren:
Beim Corona-Verfahren wird der Pulverlack oder das Pulver an einer geladenen Corona vorbeigeführt und hierbei aufgeladen, beim triboelektrischen oder elektrokinetischen Verfahren wird vom Prinzip der Reibungselektrizität Gebrauch gemacht. Auch eine Kombination von beiden Verfahren ist möglich.
Der Pulverlack oder das Pulver erhalten im Sprühgerät eine elektrostatische Aufladung, die der Ladung des Reibungspartners, im allgemeinen ein Schlauch oder Sprührohr, beispielsweise aus Polytetrafluorethylen, entgegengesetzt ist.

Als Pulverlackharze werden typischerweise Epoxidharze, carboxyl- und hydroxylgruppenhaltige Polyesterharze, Polyurethan- und Acrylharze zusammen mit den üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt.

Darüber hinaus ist gefunden worden, daß Ladungssteuermittel das Aufladungssowie das Ladungsstabilitätsverhalten von Elektretmaterialien, insbesondere Elektretfasem, erheblich verbessern können (DE-A-43 21 289). Typische Elektretmaterialien basieren auf Polyolefinen, halogenierten Polyolefinen, Polyacrylaten, Polyacrylnitrilen, Polystyrolen oder Fluorpolymeren, wie beispielsweise Polyethylen, Polypropylen, Polytetrafluorethylen und perfluoriertes Ethylen und Propylen, oder auf Polyestern, Polycarbonaten, Polyamiden, Polyimiden, Polyetherketonen, auf Polyarylensulfiden, insbesondere Polyphenylensulfiden, auf Polyacetalen, Celluloseestern, Polyalkylenterephthalaten sowie Mischungen daraus. Elektretmaterialien, insbesondere Elektretfasern, können beispielsweise zur (Feinst-)Staubfiltration eingesetzt werden. Die Elektretmaterialien können ihre Ladung durch Corona- oder Triboaufladung erhalten.

Weiterhin können Ladungssteuermittel in elektrostatischen Trennvorgängen, insbesondere in Trennvorgängen von Polymeren verwendet werden. So beschreiben Y. Higashiyama et al. (J. Electrostatics 30, pp 203 - 212 (1993)) am Beispiel des äußerlich aufgebrachten Ladungssteuermittels Trimethyl-phenyl-ammonium-tetraphenylborat, wie Polymere für Recyclingzwecke voneinander getrennt werden können. Ohne Ladungssteuermittel laden sich "Low Density Polyethylen (LDPE)" und "High Density Polyethylen" (HDPE) reibungselektrisch weitestgehend ähnlich auf. Nach Ladungssteuermittelzugabe laden sich LDPE stark positiv und HDPE stark negativ auf und lassen sich so gut trennen. Neben der äußerlichen Aufbringung der Ladungssteuermittel ist auch eine Einarbeitung derselben in das Polymer möglich, um beispielsweise ein Polymer innerhalb der triboelektrischen Spannungsreihe zu verschieben und eine entsprechende Trennwirkung zu erhalten. Ebenso lassen sich auf diese Weise andere Polymere wie z. B. Polypropylen (PP) und/oder Polyethylenterephthalat (PET) und/oder Polyvinylchlorid (PVC) voneinander trennen.

Auch Salzmineralien lassen sich trennen, wenn ihnen zuvor ein Mittel zugegeben wurde (Oberflächenkonditionierung), das die substratspezifische elektrostatische Aufladung verbessert (A. Singewald et al., Zeitschrift für Physikal. Chem., Bd. 124, S. 223 - 248 (1981)).

Weiterhin werden Ladungssteuermittel als "Electroconductivity Providing Agents" (ECPA) (JP-05-163 449) in Tinten für Tintenstrahldrucker sowie für "electronic inks" eingesetzt.

Aus der DE-A1-39 33 166 ist ein Silika-Feinpulver, das mit einem speziellen Polysiloxan behandelt wurde, als Entwickler für Bilderzeugungsverfahren bekannt. In der EP-A1-0 575 805 ist eine Ladungssteuermittel-Zusammensetzung beschrieben, die eine Feststoffmischung aus einem quartären Ammoniumsalz und einem anorganischen Pigment, wie z.B. Ca-Sulfat oder Ca-Silikat, ist.

Die DE-A-31 20 542 offenbart die Verwendung von rein anorganischen salzartigen Struktursilikaten als Ladungssteuermittel in Tonern.
Die JP 08 006295 offenbart die Verwendung von quartären Ammoniumsalzen von Polyanionen als Ladungssteuermittel in Tonern.
Die US-A-4,808,849 offenbart die Verwendung von Phyllosilikat-Mineralien in Elektretmaterialien.

Aufgabe der vorliegenden Erfindung war es, wirksame und ökotoxikologisch verträgliche Ladungssteuermittel zu finden, die insbesondere eine hohe Schnellaufladung aufweisen. Weiterhin sollten sie in verschiedenen praxisnahen Tonerbindemitteln wie Polyestern, Polystyrolacrylaten oder Polystyrolbutadienen/Epoxidharzen sowie Cycloolefincopolymeren gut und unzersetzt dispergierbar sein. Weiterhin sollte ihre Wirkung weitgehend unabhängig von der Harz/Carrier-Kombination sein, um eine breite Anwendung zu erschließen.

Ebenso sollten sie in gängigen Pulverlack-Bindemitteln und Elektretmaterialien wie z. B. Polyester (PES), Epoxid, PES-Epoxyhybrid, Polyurethan, Acrylsystemen sowie Polypropylenen gut und unzersetzt dispergierbar sein.

Bezüglich ihrer elektrostatischen Effizienz sollten die Ladungssteuermittel bereits bei möglichst geringer Konzentration (1 % oder kleiner) wirksam sein und diese Effizienz in Verbindung mit Ruß oder anderen Farbmitteln nicht verlieren. Von Farbmitteln ist bekannt, daß sie die triboelektrische Aufladung von Tonern teilweise nachhaltig beeinflussen können.

Überraschenderweise hat sich nun gezeigt, dass nachstehend beschriebene salzartige Struktursilikate vorteilhafte Ladungssteuereigenschaften und hohe Thermostabilitäten besitzen, wobei die Ladungssteuereigenschaft weder durch Kombination mit Ruß oder anderen Farbmitteln verloren geht. Darüber hinaus sind die Verbindungen mit den üblichen Toner-, Pulverlack- und Elektretbindemitteln gut verträglich und lassen sich leicht dispergieren. Weiterhin können die üblicherweise negativ steuernden Harz-Carrier-Systeme wirkungsvoll auch positiv aufgeladen werden.

Gegenstand der vorliegenden Erfindung ist die Verwendung von salzartigen Struktursilikaten, worin ein niedermolekulares organisches Kation enthalten ist, welches ein substituiertes Ammonium-, Phosphonium-, Thionium-, Triphenylcarbonium-Ion oder ein kationischer Metallkomplex ist, und das Anion ein Insel-, Ring-, Gruppen-, Ketten-, Bänder-, Schicht- oder Gerüstsilikat oder eine Kombination davon ist, als Ladungssteuermittel in elektrophotographischen Tonern und Entwicklern, in Pulverlacken, Elektretmaterialien und in elektrostatischen Trennverfahren.

Nach üblicher Definition liegen den genannten Struktursilikaten folgende Bruttoformeln zugrunde:
für Inselsilikate [SiO₄]⁴⁻, für Gruppensilikate [Si₂O₇]⁶⁻, für Ringsilikate [SiO₃]ₙ²⁻,
für Kettensilikate [SiO₃]ₘ²⁻, für Bandsilikate [Si₄O₁₁]ₘ⁶⁻, für Schichtsilikate [Si₂O₅]ₘ²⁻
und für Gerüstsilikate [AlₐSi₁₋ₐO₂]ₘ^{a-}, wobei n = 3, 4, 6 oder 8, m ganzzahlig und ≥1
und 0 < a < 1 ist. Struktursilikate werden häufig von weiteren niedermolekularen Anionen begleitet, wie z.B. OH⁻, F⁻, Cl⁻, Br⁻, J⁻, BO₃³⁻, BO₂(OH)²⁻, BO(OH)₂⁻, HCO₃⁻, CO₃²⁻ , NO₃⁻, HSO₄⁻, SO₄²⁻, H₂PO₄⁻, HPO₄²⁻, PO₄³⁻, HS⁻, S²⁻.
Ferner können in Struktursilikaten einzelne Si-Atome teilweise durch andere Atome, wie z.B. Al, B, P oder Be, substituiert sein ("Alumosilikate", "Borosilikate" usw.). Natürlich vorkommende oder synthetisch hergestellte Struktursilikate zeichnen sich weiterhin dadurch aus, dass sie ein oder mehrere verschiedene Kationen enthalten, die oftmals leicht austauschbar sind, wie z.B. Na⁺, K⁺, Mg²⁺, Ca²⁺, und z.B. durch organische lonen ersetzt werden können, wobei sich ihr chemisches und physikalisches Verhalten ändern können. Das so veränderte Silikat kann z.B. stark hydrophobisiert und damit in unpolaren Medien gut verarbeitbar sein. Bei Schichtsilikaten wird auf diese Weise das einzelne Silikatplättchen durch die organischen lonen umhüllt. Diese gecoateten Moleküle können sich über ihre Flächen zu Lamellen zusammenlagem. Bei Verwendung eines Überschusses an organischen lonen können diese auch zusätzlich zwischen den Lamellen eingelagert werden.

Bevorzugte Struktursilikate im Sinne der vorliegenden Erfindung sind Montmorillonit, Bentonit, Hectorit, Kaolinit, Serpentin, Talk, Pyrophyllit, Glimmer, Phlogopit, Biotit, Muscovit, Paragonit, Vermiculit, Beidellit, Xantophyllit, Margarit, Feldspat, Zeolith, Wollastonit, Aktinolith, Amosit, Krokydolith, Sillimanit, Nontronit, Smectit, Sepiolith, Saponit, Faujasith, Permutit und Sasil.

Beispiele für natürlich vorkommende Struktursilikate sind:
Be₂[SiO₄] Phenakit, Forsterit Mg₂[SiO₄], Olivin (Mg,Fe)₂[SiO₄], Fayalit Fe₂[SiO₄], Granate M₂^{III}M₃^{II} [SiO₄]₃ (M^{II}=Mg²⁺, Ca²⁺, Fe²⁺, Mn²⁺, M^{III} = Al³⁺, Fe³⁺, Cr³⁺), Zirkon Zr[SiO₄], Thortveitit Sc₂[Si₂O₇], Barysilit Pb₃[Si₂O₇], Hemimorphit Zn₄(OH)₂ [Si₂O₇], α-Wollastonit Ca₃ [Si₃O₉], Benitoit BaTi [Si_{3O9}], Beryll Al₂Be₃ [Si₆O₁₈], Dioptas Cu₆ [Si₆O₁₈]⁻⁶ H₂O, Dravit Na{Mg₃Al₆(OH)₄(BO₃)₃[Si₆O₁₈]}, Schörl Na{Fe3^{II}(Al,Fe^{III})₆(OH)₄(BO₃)₃[Si₆O₁₈]}, β-Wollastonit Ca[SiO₃], Enstatit Mg[SiO₃], Diopsid CaMg[SiO₃]₂, Spodumen LiAl[SiO₃]₂, Pyroxene, Amphibole, Tremolit Ca₂Mg₅(OH)₂[Si₄O₁₁]₂, Anthophyllit (Mg,Fe^{II})₇(OH)₂[Si₄O₁₁]₂, Aktinolith (Ca,Na)₂(Fe,Mg,Al)₅(OH)₂[(Si,Al)₄O₁₁]₂, Amosit (Fe^{II}Mg,Al)₇(OH)₂[(Si,Al)₄O₁₁]₂, Krokydolith Na₂(Fe^{II},Mg)₃(Fe^{III})₂[(Si,Al)₄O₁₁]₂, Sillimanit Al[AlSiO₅], Mullit, Krauskopfit, Rhodonit, Stokesit, Serpentin Mg₃(OH)₄[Si₂O₅], Kaolinit Al₂(OH)₄[Si₂O₅], Halloysit Al₂(OH)₄[Si₂O₅]-2 H₂O, Kaolin, Petalit LiAl[Si₂O₅]₂, Apophyllit Ca₄K(F)[Si₂O₅]_{4,} Gillespit BaFe[Si₂O₅]₂, Anorthit Ca₂[SiAlO₄]₄, Hexacelsian Ba₂[SiAlO₄]₄, Talk Mg₃(OH)₂[Si₂O₅]₂, Pyrophyllit Al₂(OH)₂[Si₂O₅]₂; Kanemit NaH[Si₂O₅]; Alumoschichtsilikate: Glimmer, Phlogopit K{Mg₃(OH,F)₂[AlSi₃O₁₀]}, Biotit K{(Mg,Fe,Mn)₃(OH,F)₂[AlSi₃O₁₀]}, Paragonit Na{Al₂(OH,F)₂[AlSi₃O₁₀]}, Muskovit K{Al₂(OH,F)₂[AlSi₃O₁₀]}, Fluormuskovit K{Al₂F₂[AlSi₃O₁₀]}, Glimmer der Zusammensetzung (K,H₃O)_{y}{Mg₃(OH)₂[Si₄-yAl_{y}O₁₀]} oder (K,H₃O)_{y}{Al₂(OH)₂-[Si_{4-y}Al_{y}O₁₀]}, wobei y = 0,7 bis 0,9, Sprödglimmer, z.B. Xantophyllit Ca{Mg₃(OH)₂[Al₂Si₂O₁₀]} oder Margarit Ca{Al₂(OH)₂[Al₂Si₂O₁₀]}, glimmerartige Silikate wie z.B. Vermiculit (Mg(H₂O)₆•2 H₂O)_{0,66}{[Mg,Fe^{III},Al)₃ (OH)₂[Al₁,₂₅Si_{2,75}O₁₀]}, Illite, Montmorillonit Na_{0.33}{(Al_{1,67} Mg_{0,33})(OH)₂[Si₄O₁₀]},
Bentonite, Beidellit (Ca,Na)_{0,3}{Al₂(OH)2[Al_{0,5}Si_{3,5}O₁₀]}, Nontronit Na_{0,33} {Fe₂^{III}(OH)₂[Al_{0,33} Si_{3,67}O₁₀]}, Sepiolith, Smectite, Saponit (Ca,Na)_{0,33}{(Mg,Fe^{II})₃(OH)₂[Al_{0,33}Si_{3,67}O₁₀]}, Laponit oder Hectorit Na_{0,33} {(Mg,Li)₃(OH,F)₂[Si₄O₁₀]}, Feldspate wie z.B. K[AlSi₃O₈], Na[AlSi₃O₈], Ca[Al₂Si₂O₈], Na[AlSiO₄], K[AlSi₂O₆];
Alumogerüstsilikate wie z.B. Zeolithe, beispielsweise Faujasit Na₂Ca[Al₂Si₄O₁₂]₂•16 H₂O, Chabasit (Na₂,Ca)[Al₂Si₄O₁₂]•6 H₂O, Mordenit Na₂[Al₂Si₁₀O₂₄]•6 H₂O, Natrolith Na₂[Al₂Si₃O₁₀]•2 H₂O, Permutit, Sasil, Zeolith A Na₁₂[Al₁₂Si₁₂O₂₄]•27 H₂O, Zeolith X Na₄₃[Al₄₃Si₈₃O₁₂₆]•132 H₂O, Zeolith Y Na₂₈[Al₂₈Si₆₈O₉₆]•125 H₂O, andere Alumogerüstsilikate, wie z.B. Ultramarine oder Lasurit.

Das ionische Struktursilikat kann sowohl natürlichen Ursprungs sein, z.B. in oder neben einem natürlich vorkommenden Mineral oder Gestein enthalten sein, wie beispielsweise Bentonit oder Montmorillonit, als auch ein synthetisch hergestelltes Struktursilikat sein, z.B. ein Magnesiumhydrosilikat oder ein synthetisches Hectorit (z.B. DE-A-2718 576) oder Na₂[Si₂O₅].
Bei einem natürlich vorkommenden Struktursilikat kann die geografische Lagerstätte Einfluß auf die chemischen und physikalischen Eigenschaften des Materials haben. lonische Struktursilikate, welche in der Natur oftmals von anderen Mineralien oder Gesteinen begleitet werden (z.B. Quarz), können durch mechanische oder chemische Verfahrensschritte aufgearbeitet sein, beispielsweise feinstgemahlen, von anderen Begleitsubstanzen gereinigt oder separiert, pH-behandelt, dehydratisiert, druckbehandelt, thermisch behandelt, oxidativ oder reduktiv oder mit chemischen Hilfsstoffen behandelt sein.

Handelsnamen für im Sinne der Erfindung einsetzbare Struktursilikate sind: ®Tonsil, ®Granosil, ®Südflock, ®Copisil, ®Opazil, ®Printosil, ®Lightcoat, ®Jetsil, ®Geko, ®Ecosil, ®Tixoton, ®Bentonil, ®Montigel, ®Calcigel, ®Calrit, ®Laundrosil, ®Bionit, ®Edasil, ®Agriben, ®Tixogel, ®Optibent, ®Optigel, ®Airsec, ®Albion Kaolin, ®Biokat's, ®Container Dri, ®Desi Pak, ®Ivyblock, ®Montigel, ®Detbuild, ®Bleach, ®Volclay, ®Bentobrite, ®Polargel, ®Suspengel.
Einsetzbar im Sinne der Erfindung sind auch Struktursilikate, die auch in anderen Bereichen Anwendung finden, wie beispielsweise Bleichbentonit, Papierbentonite, Gießereibentonite, Keramikbentonite, Trockenmittel, Verdickungsmittel, Antiabsetzmittel, Katalysatoren, Wasserenthärtungs-/Wasseraufbereitungs- und -reinigungsmittel.

Bevorzugt sind niedermolekulare, d.h. nicht-polymere, Ammoniumionen der Formeln (a)-(j): 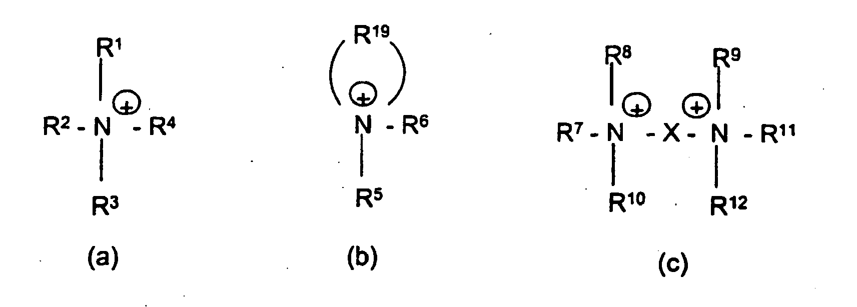 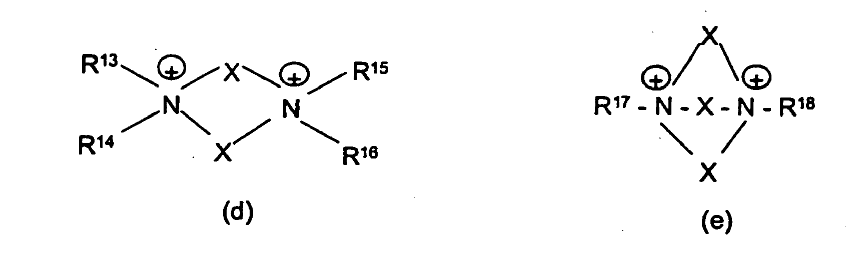 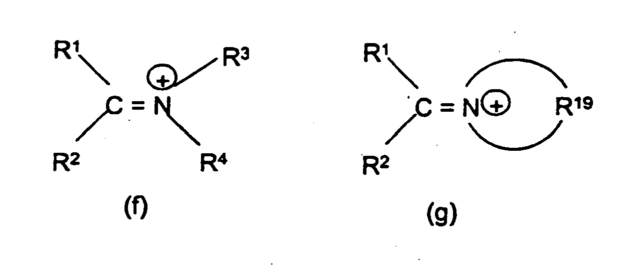 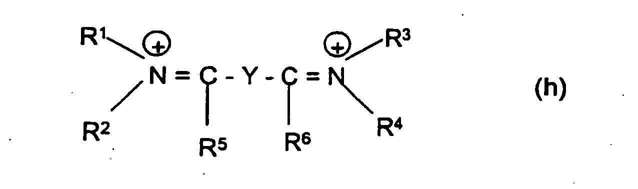 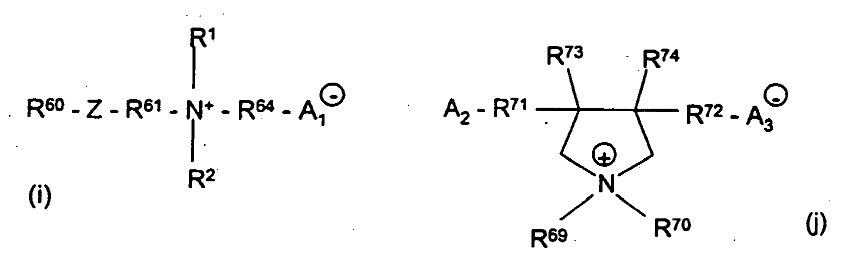 worin
R¹ bis R¹⁸ gleich oder verschieden sind und für Wasserstoff, CN, (CH₂)₁₋₁₈CN, Halogen, z.B. F, Cl oder Br, verzweigtes oder unverzweigtes C₁-C₃₂-Alkyl, ein- oder mehrfach ungesättigtes C₂-C₃₂-Alkenyl, insbesondere C₂-C₂₂-Alkenyl, wie z.B. Talgfettalkyl; C₁-C₂₂-Alkoxy, C₁-C₂₂-Hydroxyalkyl, C₁-C₂₂-Halogenalkyl, C₂-C₂₂-Halogenalkenyl, C₁-C₂₂-Aminoalkyl, (C₁-C₁₂)-Trialkyl-ammonium-(C₁-C₂₂)-alkyl; (C₁-C₂₂)-Alkylen-(C=O)O-(C₁-C₃₂)alkyl, (C₁-C₂₂)-Alkylen-(C=O)O-aryl, (C₁-C₂₂)-Alkylen-(C=O)NH-(C₁-C₃₂)alkyl, (C₁-C₂₂)-Alkylen-(C=O)NH-aryl, (C₁-C₂₂)-Alkylen-O(C=O)-(C₁-C₃₂)alkyl, insbesondere (C₁-C₁₈)Alkylen-O(CO)-(C₁-C₃₂)alkyl, (C₁-C₂₂)-Alkylen-O(CO)aryl, (C₁-C₂₂)Alkylen-NH(C=O)-(C₁₋C₃₂₎alkyl, (C₁-C₂₂)-Alkylen-NHCO-aryl,
wobei in die Säureester- oder Säureamidbindungen 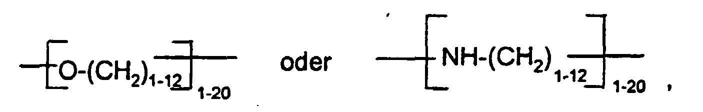 eingeschoben sein kann;
[(C₁-C₁₂)alkylen-O-]₁₋₁₀₀-H; Aryl, (C₁-C₁₈)-Alkylenaryl, -(O-SiR'₂)₁₋₃₂,O-SiR'₃, wobei R' die Bedeutung C₁-C₁₂-Alkyl, Phenyl, Benzyl oder C₁-C₁₂-Alkoxy hat; Heterocyclus, C₁-C₁₈-Alkylen-heterocyclus;
R¹⁹ für C₄-C₁₁-Alkylen, -(C₂H₄-O-)₁₋₁₇-(CH₂)₁₋₂-, -(C₂H₄-NR-)₁₋₁₇-(CH₂)₁₋₂-, wobei R Wasserstoff oder C₁-C₁₂-Alkyl ist;
X die Bedeutung von Y sowie -CO-CH₂-CO-, 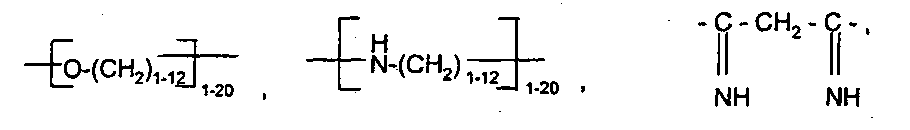 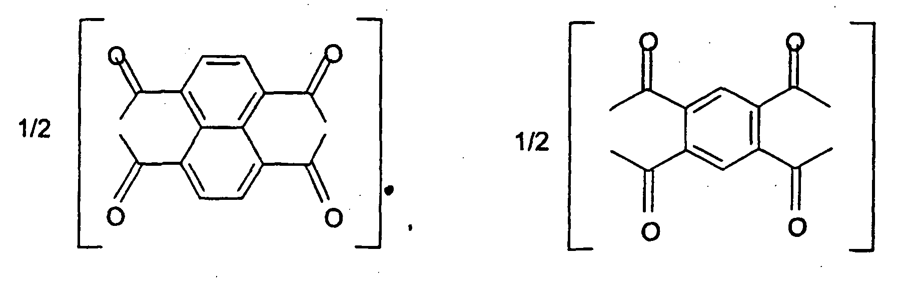 oder 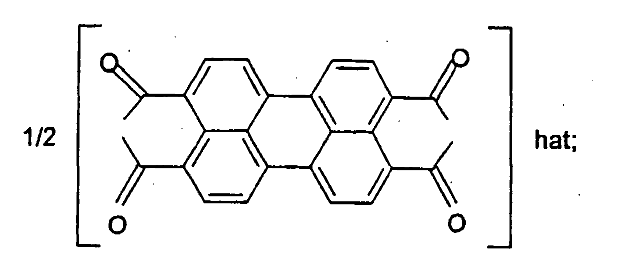 Y die Bedeutung 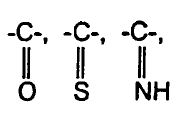 -(CH₂)₁₋₁₈-, 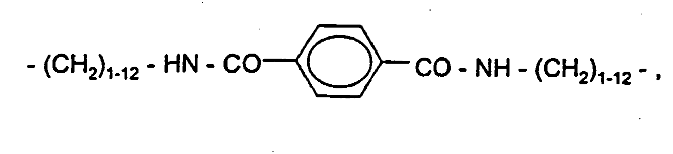 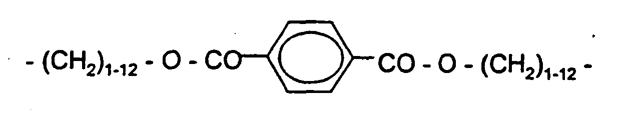 oder o-, p-, m-(C₆-C₁₄)-Arylen oder (C₄-C₁₄)-Heteroarylen mit 1, 2, 3 oder 4 Heteroatomen aus der Gruppe N, O und/oder S hat;
- R⁶⁰: für C₁-C₃₂-Acyl, C₁-C₂₂-Alkyl, C₂-C₂₂-Alkenyl, C₁-C₁₈-Alkylen-C₆-C₁₀-aryl, C₁-C₂₂-Alkylen-heterocyclus, C₆-C₁₀-Aryl oder (C₄-C₁₄)-Heteroaryl mit 1, 2, 3 oder 4 Heteroatomen aus der Gruppe N, O und/oder S,
- R⁶¹ und R⁶⁴: für -(CH₂)₁₋₁₈-, C₁-C₁₂-Alkylen-C₆-C₁₀-arylen, C₆-C₁₀-Arylen, C₀-C₁₂-Alkylen-heterocyclus;
Z für -NH- oder -O- ;
A₁^{θ} und A₃^{θ} für -COO^{θ}, -SO₃^{θ}, -OSO₃^{θ}, -SO₂^{θ}, -COS^{θ} oder -CS₂^{θ};
A₂ für -SO₂Na, -SO₃Na, -SO₂H, -SO₃H oder Wasserstoff;
R⁶⁹ und R⁷⁰ unabhängig voneinander für Wasserstoff, C₁-C₃₂-Alkyl, wobei in der Alkylkette eine oder mehrere der Gruppen -NH-CO-, -CO-NH-, -CO-O- oder -O-COenthalten sein kann; C₁-C₁₈-Alkylen-aryl, C₀-C₁₈-Alkylen-heterocyclus, C₁-C₁₈-Hydroxyalkyl, C₁-C₁₈-Halogenalkyl, Aryl, -(CH₂)₃-SO₃^{θ}, 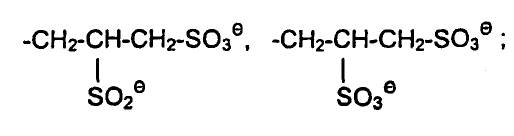 R⁷¹ und R⁷² für -(CH₂)₁₋₁₂-; und
R⁷³ und R⁷⁴ für Wasserstoff oder C₁-C₂₂-Alkyl stehen.

Soweit nicht anders beschrieben, steht "Aryl" in den vorangegangenen und nachfolgenden Definitionen vorzugsweise für C₆-C₁₈-Aryl, insbesondere Phenyl oder Naphthyl, "Heterocyclus" vorzugsweise für einen gesättigten, ungesättigten oder aromatischen, fünf- bis siebengliedrigen Ring mit 1, 2, 3 oder 4 Heteroatomen aus der Gruppe N, O und/oder S, beispielsweise für Pyridyl, Imidazolyl, Triazinyl, Pyridazyl, Pyrimidinyl, Pyrazinyl, Piperidinyl, Morpholinyl, Purinyl, Tetrazonyl, Pyrrolyl. Weiterhin können die Aryl- und Heterocyclusreste an Kohlenstoff- oder Heteroatomen einfach oder mehrfach, z.B. 2-, 3-, 4- oder 5-fach, durch C₁-C₁₂-Alkyl, C₁-C₄-Alkenyl, C₁-C₄-Alköxy, Hydroxy-(C₁-C₄)alkyl, Amino-(C₁-C₄)alkyl, C₁-C₄-Alkylimino, Carboxy, Hydroxy, Amino, Nitro, Cyano, Halogen, C₁-C₁₂-Acyl, C₁-C₄-Halogenalkyl, C₁-C₄-Alkylcarbonyl, C₁-C₄-Alkylcarbonyloxy, C₁-C₄-Alkoxycarbonyl, C₁-C₄-Alkylaminocarbonyl, C₁-C₄-Alkylcarbonylimino, C₆-C₁₀-Arylcarbonyl, Aminocarbonyl, Aminosulfonyl, C₁-C₄-Alkylaminosulfonyl, Phenyl, Naphthyl, Heteroaryl, z.B. Pyridyl, Imidazolyl, Triazinyl, Pyrimidinyl, substituiert sein.

Weiterhin bevorzugte heterocyclische Ammoniumionen sind aliphatische oder aromatische, 5 bis 12-gliedrige Heterocyclen mit 1, 2, 3 oder 4 ringangehörigen N-, O- und/oder S-Atomen, wobei 2 bis 8 Ringe annelliert sein können, insbesondere Pyridinium, Pyridazinium, Pyrimidinium, Pyrazinium, Purinium, Tetraazaporphyrinium, Piperidinium, Morpholinium, Tetrazonium. Weitere geeignete Heterocyclen sind z.B. Pyrrolium, Pyrazolium, Imidazolium, Benzimidazolium, Imidazolonium, Benzimidazolonium, Imidazolinium, Benzimidazolinium, Alkylpyrrolidino-benzimidazolonium, Indolium, Isoindolium, Indolizinium, Pyrrolizidinium, Carbazolium, Indazolium, Chinolinium, Isochinolinium, Pyrindenium, Acridinium, Phenanthridinium, Lilolinium, Julolinium, Matridinium, Cinnolinium, Chinazolinium, Chinoxalinium, Perimidinium, Phenazonium, Phenazinium, 1,10-Phenanthrolinium,
ß-Carbolinium, Chinolizinium, 1,8-Naphthyldrinium, Pteridinium, Chinuclidinium, Conidinium, Hypoxanthinium, Adeninium, Xanthinium, Isoxanthinium, Heteroxanthinium, Isoadeninium, Guaninium, Epiguaninium, Theophyllinium, Paraxanthinium, Theobrominium, Koffeinium, Isokoffeinium, Trihydroxypurinium, Porphyrinium, Tetraazaphorphyrinium, Metall-komplexiertes Tetraazaphorphyrinium (z.B. mit Mg, Ca, Sr, Ba, Al, Mn, Fe, Co, Cu, Zr, Ti,Cr, Ni, Zn),
Bis-Tetrazonium, Phenoxazinium, Aminoxanthenium, sowie an C oder Heteroatomen einfach- oder mehrfach-substituierte Derivate der genannten Kationen, wobei die Substituenten unabhängig voneinander Carboxyl, Hydroxy, C₁-C₂₂-Alkoxy, C₁-C₃₂-Alkyl, insbesondere C₁-C₂₂-Alkyl, C₂-C₂₂-Alkenyl, Hydroxy-(C₁-C₂₂)-alkyl, Amino, Aminoalkyl, C₁-C₁₈-Iminoalkyl, Alkylamido, Alkylcarbonyloxy, Alkyloxycarbonyl, (C₁-C₂₂)-Alkylen-(C=O)O-(C₁-C₃₂)alky), (C₁-C₂₂)-Alkylen-(C=O)O-aryl, (C₁-C₂₂)Alkylen-(C=O)NH-(C₁-C₃₂)alkyl, (C₁-C₂₂)Alkylen-(C=O)NH-aryl, (C₁-C₂₂)-Alkylen-O(CO)-(C₁-C₃₂)alkyl, (C₁-C₂₂)Alkylen-O(CO)-aryl, (C₁-C₂₂)Alkylen-NH(C=O)-(C₁-C₃₂)alkyl, (C₁-C₂₂)-Alkylen-NHCO-aryl; wobei in die Säureester- oder Säureamidbindungen 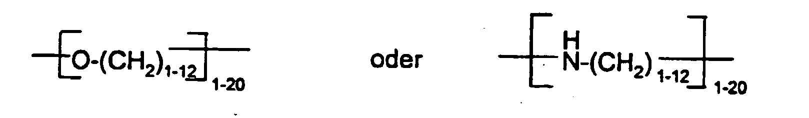 eingeschoben sein kann;
Nitro, Cyano, Halogen, Poly(C₁-C₁₂-alkylenoxid) oder C₁-C₂₂-Acyl sein können, insbesondere N- oder C-(C₁-C₂₂)-alkylierte Heterocyclen, wie vorstehend genannt, z.B. N-(C₁-C₂₀)alkyl-pyridinium oder 1-Methyl-1-stearylamidoethyl-2-stearyl-imidazolinium.

Von den Ionen der Formeln (a) - (j) sind solche von besonderem Interesse, worin R¹ bis R¹⁸ Wasserstoff, CN, CH₂-CN, CF₃, C₁-C₂₂-Alkyl, z.B. Cocosalkyl, Cetyl, Stearyl oder hydriertes Talgfettalkyl; C₂-C₂₂-Alkenyl, insbesondere C₂-C₁₈-Alkenyl, C₁-C₁₈-Alkoxy, C₁-C₁₈-Hydroxy-alkyl, C₁-C₁₈-Halogenalkyl, C₂-C₁₈-Halogenalkenyl, wobei Halogen vorzugsweise F oder Cl bedeutet, C₁-C₁₈-Aminoalkyl, (C₁-C₆)-Trialkylammonium-(C₁-C₁₈)-alkyl, (C₁-C₁₈)-Alkylen-O(C=O)-(C₁-C₂₂)alkyl, (C₁-C₁₈)-Alkylen-O(C=O)-phenyl, (C₁-C₁₈)-Alkylen-NHCO-(C₁-C₂₂)alkyl, (C₁-C₁₈)-Alkylen-NHCO-phenyl, (C₁-C₁₈-Alkylen-(C=O)O-(C₁-C₂₂)alkyl, (C₁-C₁₈)-Alkylen-(C=O)O-phenyl, (C₁-C₁₈)Alkylen-(C=O)NH-(C₁-C₂₂)alkyl, (C₁-C₁₈)-Alkylen-CONH-phenyl, Benzyl, Phenyl, Naphthyl, C₁-C₁₂-Alkylen-heterocyclus;
R¹⁹ C₄-C₅-Alkylen, -(C₂H₄-O)₁₋₉-(CH₂)₁₋₂-, -(C₂H₄-NH)₁₋₉-(CH₂)₁₋₂-;
R⁶⁰ C₁-C₁₈-Acyl, C₁-C₁₈-Alkyl, C₂-C₁₈-Alkenyl, C₁-C₁₂-Alkylen-phenyl, C₁-C₁₈-Alkylenpyridyl, Phenyl, Pyridyl;
R⁶¹ und R⁶⁴ -(CH₂)₁₋₁₂-, C₁-C₈-Alkylen-phenylen, Phenylen; C₁-C₈-Alkylenpyridylen- oder -piperidylen;
R⁷¹ und R⁷² -(CH₂)₁₋₈ und
R⁷³ und R⁷⁴ Wasserstoff oder (C₁-C₁₈)-Alkyl bedeuten.

Bevorzugte niedermolekulare organische Kationen sind weiterhin kationische Metallkomplexe, wie Metallcarboxylate, Metallsalicylate, Metallsulfonate, 1:1-Metäll-Azo-komplexe oder Metall-dithiocarbamate, wobei Metall vorzugsweise Al, Mg, Ca, Sr, Ba, TiO, VO, Cr, V, Ti, Zr, Sc, Mn, Fe, Co, Ni, Cu, Zn und ZrO ist, und der Metallkomplex gegebenenfalls ein oder mehrere weitere Liganden enthält.

Bevorzugte Metallcarboxylate und -salicylate sind solche der Formeln (k) und (I) 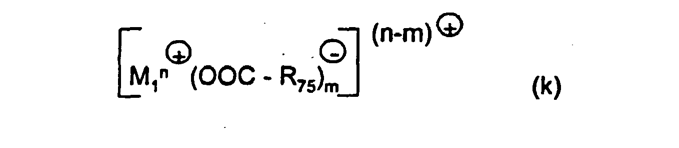 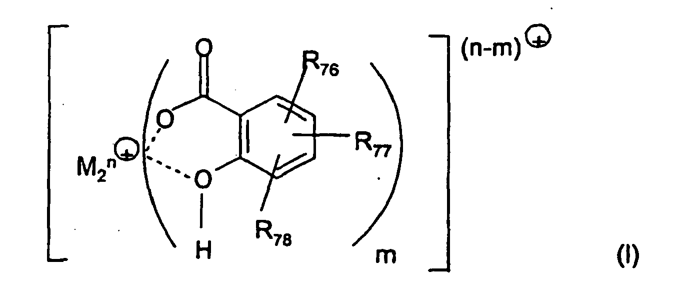 wobei n = 2, 3 oder 4;
m = 1, 2 oder 3, aber stets kleiner als n;
M₁^{n⊕} und M₂^{n⊕} unabhängig voneinander ein Metall-Kation der Hauptgruppen- oder Übergangsmetalle sind, beispielsweise B, Al, Mg, Ca, Sr, Ba, Sc, V, Ti, Zr, TiO, Cr, Mn, Fe, Co, Ni, Cu, Zn, ZrO, darstellen,
R⁷⁵ C₁-C₃₂-Alkyl (linear oder verzweigt), C₁-C₂₂-Halogenalkyl, C₁-C₁₈-Hydroxyalkyl, C₁-C₁₈-Aminoalkyl, C₁-C₁₈-Ammoniumalkyl, C₁-C₁₈-Alkylen-aryl, C₁-C₁₈-Alkylen-heterocyclus, Aryl, Heterocyclus, wie vorstehend definiert;
R⁷⁶ bis R⁷⁸ unabhängig voneinander C₁-C₁₂-Alkyl (linear oder verzweigt), C₁-C₄-Alkoxy, Hydroxy, Carboxyl, C₁-C₄-Alkenyl, Hydroxy-(C₁-C₄)-alkyl, Amino, (C₁-C₄)-Aminoalkyl, Nitro, Cyano, Halogen, C₁-C₁₂-Acyl, C₁-C₄-Iminoalkyl, C₁-C₄-Halogenalkyl, Aryl oder Heterocyclus, wie vorstehend definiert, sein können.

Weiterhin geeignet sind analoge kationische Komplexe oder Salze oben genannter Metalle mit Liganden, wie α-Hydroxyphenol, α-Aminoanilin, α-Hydroxyanilin, α-Aminobenzoesäure, Chinolin, 1,8-Diaminonaphthalin, 1,4,5,8-Tetraaminonaphthalin, 1,8-Dihydroxynaphthalin oder 1,4,5,8-Tetrahydroxynaphthalin. Weiterhin geeignet sind analoge kationische Komplexe oder Salze der oben genannten Metalle mit Liganden oder Anionen wie beispielsweise α,α-Dipyridyl, Ethylendiamin, Diethylentriamin, Triethylentetraamin, Acetylacetonat, ortho-Phenanthrolin, Benzoylketone, Ethylendi(biguanidin), Biguanidin oder Dimethylglyoxim.

### Bevorzugte 1:1-Metall-Azo-komplexe sind solche der Formeln (m) - (p)

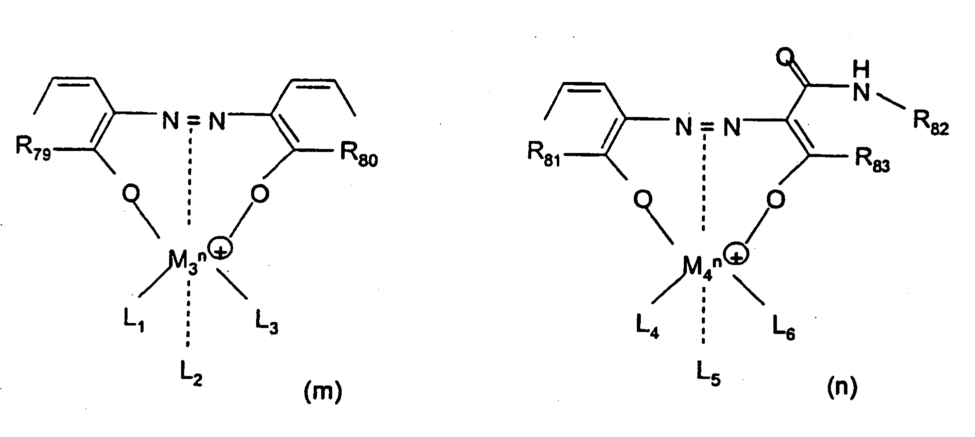

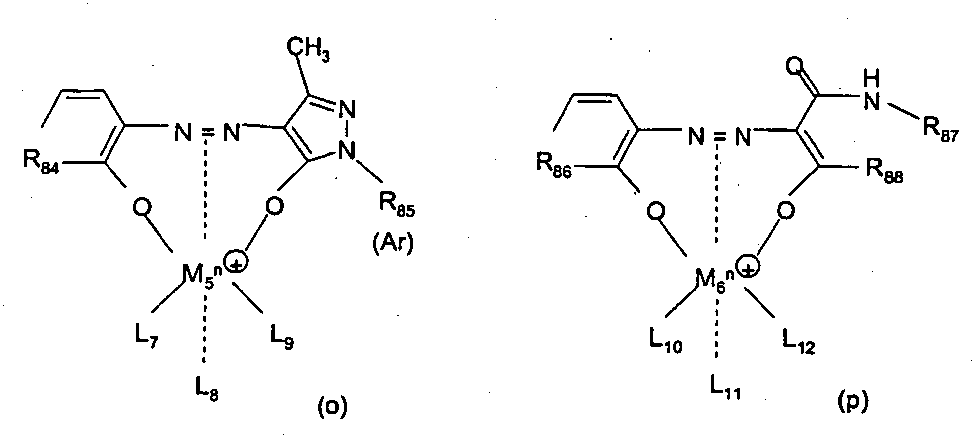

worin M₃ⁿ⁺ bis M₆ⁿ⁺ eine der Bedeutungen von M₁ⁿ⁺ oder M₂ⁿ⁺ haben,
R₇₉, R₈₀, R₈₁, R₈₄ und R₈₆, unabhängig voneinander eine zur Vervollständigung eines ein- oder zweikernigen Ringsystems aromatischen Charakters notwendige Atomgruppe sind, die gegebenenfalls Substituenten tragen kann,
R₈₂ und R₈₇ unabhängig voneinander ein gegebenenfalls Substituenten tragender Phenylrest oder ein (C₁-C₁₂)-Alkyl- oder (C₁-C₂)-Alkoxy-(C₂-C₈)-alkylrest sind,
R₈₃, R₈₅ und R₈₈ unabhängig voneinander C₁-C₁₂-Alkyl oder Phenyl, gegebenenfalls Substituenten tragend, sind,
und die Liganden L₁ bis L₁₂ unabhängig voneinander H₂O, OH⁻, NH₃, F, Cl⁻, Br⁻, J⁻, NO₃⁻, HSO₄⁻, SO₄²⁻, H₂PO₄⁻, HPO₄²⁻, P0₄³⁻, BO₃³⁻, BO₂(OH)²⁻, BO(OH)₂⁻, HCO₃⁻, CO₃²⁻, H₂S, HS⁻, S²⁻, Oxalat, Citrat, Formiat, Acetat, Propionat, Fumarat, Maleat, Tartrat,
C₁-C₄-Alkylsulfonat, Taurid, Methyltaurid, Sarcosid, Methylsarcosid, Lactat, sowie andere niedermolekulare Carboxylate und Sulfonate sein können.

Weiterhin geeignet sind kationische Komplexe oder Salze der oben genannten Metalle mit Dithiocarbamat-Liganden gemäß der Formel (o) 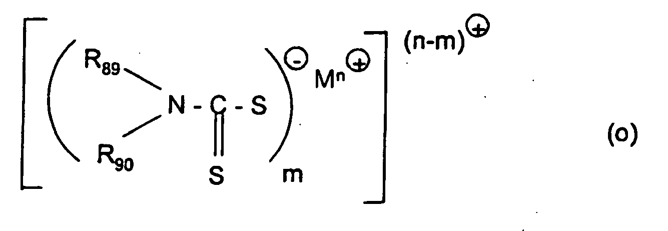 worin die Reste R₈₉ und R₉₀ unabhängig voneinander eine der Bedeutungen von R₁ haben und m und n eine Zahl von 1 bis 4 sind, wobei n > m ist.

Weiterhin geeignet sind Triaza-cyclononanium- oder Tetraaza-cyclododecanium-Kationen der allgemeinen Formeln (p) und (q) 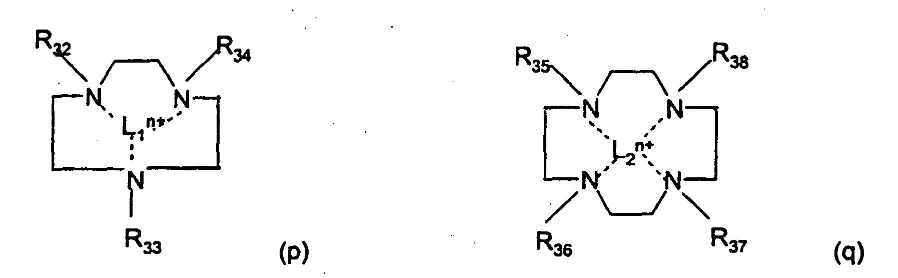 wobei R₃₂ bis R₃₈ unabhängig voneinander H, C₁-C₃₂-Alkyl, C₂-C₁₈-Alkenyl, C₁-C₁₈-Halogenalkyl, C₁-C₁₈-Alkoxy, C₁-C₁₈-Hydroxyalkyl, (C₁-C₈)Alkylen-(C₆-C₁₄)-aryl, (C₁-C₁₀)-Alkylenheteroaryl, z.B. (C₁-C₁₀)-alkylen-pyridyl, sein können, n für eine Zahl zwischen 1 und 4 steht, und L₁ und L₂ für ein niedermolekulares Kation steht, beispielsweise für Wasserstoff oder ein Hauptgruppen- oder Übergangsmetall, wie Li, Na, K, Rb, Cs, Be, Mg, Ca, Sr, Ba, Al, TiO, ZrO, Mn, VO, Fe, Co, Cu, Zn, Cr, Ni, Mo, W.

Weiterhin geeignet sind Ammonium-Kationen der allgemeinen Formel (r) 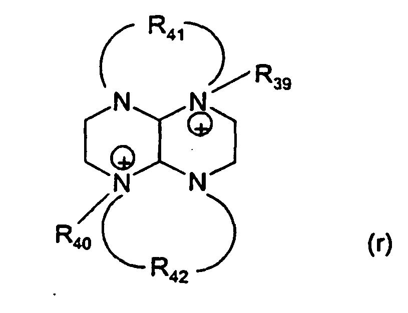 worin R₃₉ und R₄₀ unabhängig voneinander eine der Bedeutungen von R₃₂ haben; und R₄₁ und R₄₂ für (-CH₂-)ₙ stehen, wobei n = 2 bis 9 ist.
Weiterhin geeignet sind Aminosäuren der allgemeinen Formel (s) 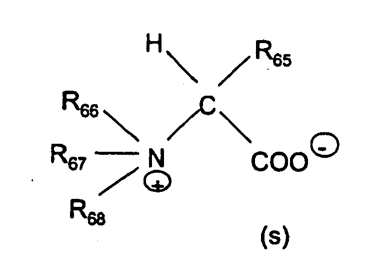 wobei die Reste R₆₆ bis R₆₈ unabhängig voneinander Wasserstoff, C₁-C₂₂-Alkyl, C₁-C₁₈-Hydroxyalkyl, (C₁-C₂₂)-Halogenalkyl, (C₁-C₁₈)-Alkylenaryl, z.B. Benzyl, (C₁-C₁₈)-Alkylenheteroaryl, (C₆-C₁₀)-Aryl, Heteroaryl, z.B. Pyridyl, Heterocyclus, z.B. Morpholinyl, Piperidinyl, (C₁-C₈)-Alkylenheterocyclus und
R₆₅ Wasserstoff, C₁-C₁₈-Alkyl, C₁-C₁₈-Hydroxyalkyl, C₁-C₁₈-Thioalkyl, C₁-C₁₈-Aminoalkyl, C₁-C₁₈-Carboxyalkyl, C₁-C₁₈-Alkylenaryl, z.B. Benzyl, C₁-C₁₈-Alkylenheteroaryl, C₁-C₁₈-Alkylenheterocyclus, C₆-C₁₀-Aryl, (C₄-C₁₀)- Heteroaryl, (C₄-C₁₀)-Heterocyclus, z.B. Morpholinyl, Piperidinyl, C₁-C₂₂-Acyl, C₁-C₁₈-Halogenalkyl, Cyano, sein können.

Weiterhin geeignet sind Triphenylmethan-Kationen der Formel 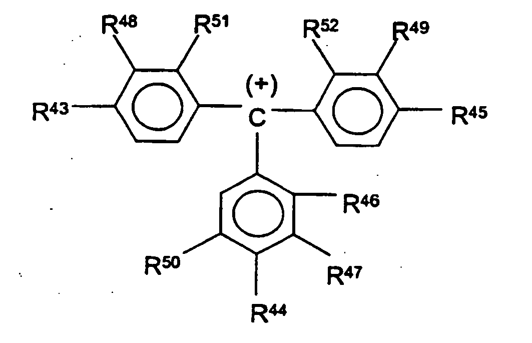 worin
R⁴³ und R⁴⁵ gleich oder verschieden sind und -NH₂, eine Mono- und Dialkylaminogruppe, deren Alkylgruppen 1 bis 4, vorzugsweise 1 oder 2, C-Atome haben, eine Mono- oder Di-omega-hydroxyalkylaminogruppe, deren Alkylgruppen 2 bis 4, vorzugsweise 2, C-Atome haben, eine gegebenenfalls N-(C₁-C₄)Alkylsubstituierte Phenyl- oder Phenalkylaminogruppe, deren Alkyl 1 bis 4, vorzugsweise 1 oder 2, C-Atome hat und deren Phenylkern einen oder zwei der Reste Methyl, Ethyl, Methoxy, Ethoxy, Sulfo tragen kann, bedeuten,
R⁴⁴ Wasserstoff ist oder eine der für R⁴³ und R⁴⁵ genannten Bedeutungen hat,
R⁴⁶ und R⁴⁷ Wasserstoff, Halogen, vorzugsweise Chlor, oder eine Sulfonsäuregruppe bedeuten oder R⁴⁶ mit R⁴⁷ zusammen einen ankondensierten Phenylring bilden,
R⁴⁸, R⁴⁹, R⁵¹ und R⁵² jeweils Wasserstoff oder einen Alkylrest mit 1 oder 2 C-Atomen, vorzugsweise Methyl, bedeuten und
R⁵⁰ Wasserstoff oder Halogen, vorzugsweise Chlor, ist.

Weiterhin geeignet sind Phosphonium- und Thionium-Kationen der Formeln (t) und (u) 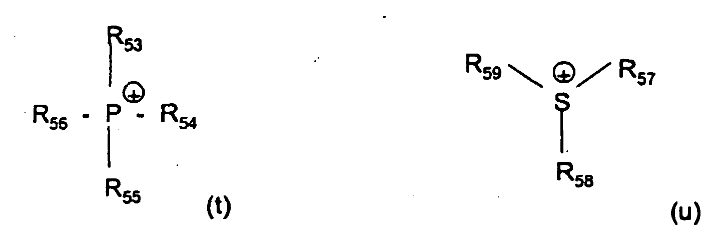 worin R₅₃ bis R₅₉ unabhängig voneinander C₁-C₁₈-Alkyl, C₂-C₁₈-Alkenyl, C₁-C₁₈-Alkoxy, C₁-C₁₈-Hydroxyalkyl, (C₁-C₈)Alkylen-(C₆-C₁₀)aryl, z.B. Benzyl, Alkylenheteroaryl, C₆-C₁₀-Aryl, Heteroaryl, z.B. Pyridinyl, sind.

Besonders bevorzugt sind fluorierte Ammoniumionen der Formel (x) 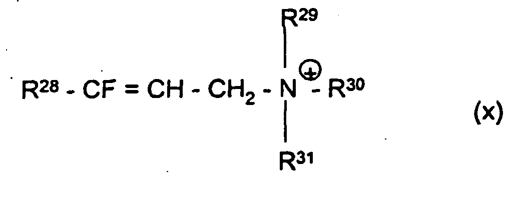 worin
R²⁸ perfluoriertes Alkyl mit 5 bis 11 C-Atomen,
R²⁹, R³⁰ und R³¹ gleich oder verschieden sind und Alkyl mit 1 bis 5 C-Atomen, vorzugsweise 1 bis 2 C-Atomen, bedeuten.

Die ein niedermolekulares organisches Kation enthaltenden Struktursilikate können hergestellt werden, indem man ein oder mehrere natürliche oder synthetische Struktürsilikate mit einem das niedermolekulare organische Kation enthaltenden Salz, z.B. das entsprechende Chlorid, Bromid, lodid, Methylsulfat, in wäßriger Suspension, zweckmäßig bei einem pH-Wert zwischen 0 und 14, vorzugsweise zwischen 1 und 13, zweckmäßig bei einer Temperatur von 0 bis 160°C, vorzugsweise von 5 bis 140°C, zweckmäßig bei einem Druck von 1 bis 20 bar, für 5 Minuten bis 48 Stunden, vorzugsweise 10 Minuten bis 24 Stunden, in einem molaren Verhältnis organisches Kation : Silikat von 1:100 bis 10:1, vorzugsweise von 1:20 bis 3:1, zusammengibt. Es ist vorteilhaft, das Struktursilikat zwischen ½ und 48 Stunden, vorzugsweise zwischen 1 und 24 Stunden, z.B. bei einer Temperatur von 5 bis 100°C, in Wasser vorzudispergieren. Es ist weiterhin vorteilhaft, das Salz des organischen Kations und/oder die wässrige Suspension des/der Struktursilikate(s) vor der Umsetzung in wäßrigem Medium auf einen pH zwischen 1 und 12, vorzugsweise 5 und 10 einzustellen.

Ein salzartiges Struktursilikat, worin das Silikat Hectorit, Beidellit, Illit, Muskovit, Xantophyllit, Margarit, Sepiolith, Saponit, Glimmer, Feldspat, Nontronit, Montmorillonit, Smectit, Bentonit, Faujasit, Zeolith A, X oder Y, Permutit, Sasil oder eine Kombination davon; und das Kation ein Ion der vorstehend beschriebenen Formel (x) ist, ist neu und ebenfalls Gegenstand der vorliegenden Erfindung. Die Herstellung dieser neuen Verbindungen kann wie vorstehend beschrieben erfolgen. Diese Verbindungen können überraschenderweise im eingesetzten Medium einen Charge Assistent Effect zeigen, insbesondere eine unterstützende Anti-Offset-Wirkung erreichen (bessere Ablösung des Toners von beweglichen Teilen der Druckmechanik, die mit dem Toner in Berührung kommen, z.B. Photoleiter, Umlenkrollen).

Die erfindungsgemäß verwendeten Salze ionischer Struktursilikate können genau auf das jeweilige Harz/Toner-System abgestimmt werden. Ein weiterer technischer Vorteil dieser Verbindungen liegt darin, daß sie sich gegenüber den unterschiedlichen Bindemittelsystemen inert verhalten und somit vielfältig einsetzbar sind, wobei von besonderer Bedeutung ist, daß sie nicht in der Polymermatrix gelöst sind, sondern als kleine, feinstverteilte Festkörper vorliegen. Ferner zeigen sie hohe und meist konstante Ladungssteuereigenschaften sowie gute Thermostabilitäten. Weiterhin sind die erfindungsgemäß eingesetzten Struktursilikate rieselfähig und besitzen eine gute Dispergierbarkeit.

Dispergierung bedeutet die Verteilung eines Stoffes in einem anderen, im Sinne der Erfindung die Verteilung eines Ladungssteuermittels im Tonerbindemittel, Pulverlackbindemittel oder Elektretmaterial.
Es ist bekannt, daß kristalline Stoffe in ihrer gröbsten Form als Agglomerate vorliegen. Um eine homogene Verteilung im Bindemittel zu erreichen, müssen diese durch den Dispergiervorgang in kleinere Aggregate oder idealerweise in Primärteilchen zerteilt werden. Die Ladungssteuermittel-Partikel, die nach der Dispergierung im Bindemittel vorliegen, sollten kleiner als 1 µm, vorzugsweise kleiner als 0,5 µm, sein, wobei eine enge Teilchengrößen-Verteilung von Vorteil ist.
Für die Teilchengröße, definiert durch den d₅₀-Wert, finden sich stoffabhängig optimale Wirkbereiche. So sind beispielsweise grobe Teilchen (1 mm) zum Teil gar nicht oder nur mit einem erheblichen Zeit- und Energieaufwand dispergierbar, während sehr feine Teilchen im Submicron-Bereich ein erhöhtes sicherheitstechnisches Risiko, wie die Möglichkeit der Staubexplosion, bergen.

Die Teilchengröße und Form wird entweder durch die Synthese und/oder Nachbehandlung eingestellt und modifiziert. Häufig wird die geforderte Eigenschaft erst durch gezielte Nachbehandlung wie Mahlung und/oder Trocknung möglich. Hierzu bieten sich verschiedene Mahltechniken an. Vorteilhaft sind beispielsweise Luftstrahlmühlen, Schneidmühlen, Hammermühlen, Perlmühlen sowie Prallmühlen.

Typischerweise handelt es sich bei den in der vorliegenden Erfindung erwähnten Bindemittelsystemen um hydrophobe Materialien. Hohe Wassergehalte des Ladungssteuermittels können entweder einer Benetzung entgegenstehen oder aber eine Dispergierung begünstigen (Flushen). Daher ist der praktikable Feuchtegehalt stoffspezifisch.

Die erfindungsgemäßen Verbindungen sind durch folgende chemisch/physikalische Eigenschaften gekennzeichnet:
Der Wassergehalt, nach der Karl-Fischer Methode bestimmt, liegt zwischen 0,001 % und 30 %, bevorzugt zwischen 0,01 und 25 % und besonders bevorzugt zwischen 0,1 und 15 %, wobei das Wasser adsorbiert und/oder gebunden sein kann, und sich dessen Anteil durch Temperatureinwirkung bis 200°C und Vakuum bis 10⁻⁸ Torr oder durch Wasserzugabe oder Lagerung unter definierten Luftfeuchtebedingungen einstellen läßt.
Überraschenderweise zeigen die erfindungsgemäßen Verbindungen, die ein oder mehrere vorstehend definierte organische Kationen enthalten, nach 48 h Lagerung bei 90 % rel. Luftfeuchte und 25°C in einem Klimaprüfschrank keinen sonderlich erhöhten H₂O-Gehalt (Karl-Fischer Methode), während die analogen Struktursilikate mit Metallkationen deutlich höhere H₂O-Gehalte aufweisen, teilweise ein Vielfaches gegenüber den vor der Klimalagerung.

Die Teilchengröße, mittels lichtmikroskopischer Auswertung oder Laserlichtbeugung bestimmt, und definiert durch den d₅₀-Wert, liegt zwischen 0,01 µm und 1000 µm, bevorzugt zwischen 0,1 und 500 µm und ganz besonders bevorzugt zwischen 0,5 und 400 µm. Besonders vorteilhaft ist es, wenn durch die Mahlung eine enge Teilchengröße resultiert. Bevorzugt ist ein Bereich Δ (d₉₅-d₅₀) von kleiner als 500 µm, insbesondere kleiner als 400 µm.

Die Leitfähigkeit der 5 %igen wässrigen Dispersion liegt zwischen 0,001 und 2000 mS, vorzugsweise zwischen 0,01 und 100 mS. Die erfindungsgemäßen Verbindungen enthalten sowohl kristalline als auch amorphe Anteile.
Die erfindungsgemäß verwendeten Verbindungen, eingearbeitet in ein Tonerbindemittel, zeigen in einer thermischen Gradientenprüfung (Kofler-Test) eine Temperaturstabilität bis 200°C (keine Verfärbung).
Bei der elektrokinetischen Oberflächenpotentialbestimmung mittels SCD (streaming current detection) zeigen die erfindungsgemäßen Verbindungen mit vorstehend definierten organischen Kationen überraschenderweise deutlich niedrigere Oberflächenpotentiale (positives oder negatives Vorzeichen) als die entsprechenden Struktursilikate mit Metallkationen. Bei der Titration dieser Verbindungen mit entsprechenden oberflächenaktiven Reagenzien bis zum Nullpunkt des Oberflächenpotentials (SCD-Verfolgung der Titration ) ist bei den Verbindungen mit Metallkationen deutlich mehr oberflächenaktives Reagenz nötig als bei den entsprechenden Struktursilikaten mit organischen Kationen. Dies weist auf eine hohe Stabilität der Salzbindung zwischen Struktursilikat und organischem Kation hin.

Die erfindungsgemäß eingesetzten Salze ionischer Struktursilikate können auch mit weiteren positiv oder negativ steuernden Ladungssteuemlifteln kombiniert werden, um gute anwendungstechnische Aufladbarkeiten zu erzielen, wobei die GesamtKonzentration der Ladungssteuermittel zweckmäßig zwischen 0,01 und 50 Gew.-%, bevorzugt zwischen 0,05 und 20 Gew.-%, besonders bevorzugt zwischen 0,1 und 5 Gew.-%, liegt, bezogen auf das Gesamtgewicht des elektrophotographischen Toners, Entwicklers, Pulvers oder Pulverlacks.

Als weitere Ladungssteuermittel kommen beispielsweise in Betracht:
Triphenylmethane; Ammonium- und Immoniumverbindungen, Iminiumverbindungen; fluorierte Ammonium- und fluorierte Immoniumverbindungen; biskationische Säureamide; polymere Ammoniumverbindungen; Diallylammoniumverbindungen; Arylsulfid-Derivate, Phenolderivate; Phosphoniumverbindungen und fluorierte Phosphoniumverbindungen; Calix(n)arene, ringförmig verknüpfte Oligosaccharide (Cyclodextrine) und deren Derivate, insbesondere Borester-Derivate, Interpolyelektrolytkomplexe (IPECs); Polyestersatze; Metallkomplexverbindungen, insbesondere Salicylat-Metall-Komplexe und Salicylat-Nichtmetalkomplexe, Hydroxycarbonsäure-Metall-Komplexe und Hydroxycarbonsäure-Nichtmetallkomplexe, Benzimidazolone; Azine, Thiazine oder Oxazine, die im Colour Index als Pigments, Solvent Dyes, Basic Dyes oder Acid Dyes aufgeführt sind.

Besonders bevorzugt sind die nachfolgend genannten Ladungssteuermittel, die einzeln oder in Kombination miteinander mit den Salzen der ionischen Struktursilikate kombiniert werden können:
Triphenylmethane, wie z.B. beschrieben in US-A-5 051 585;
Ammonium- und Immoniumverbindungen, wie z.B. beschrieben in US-A-5 015 676; Fluorierte Ammonium- und fluorierte Immoniumverbindungen, wie z.B. beschrieben in US-A-5 069 994; biskationische Säureamide, wie z.B. beschrieben in
WO 91/10172; Diallylamnioniumverbindungen, wie z.B. beschrieben in
DE-A-4 142 541, DE-A-4 029 652 oder DE-A-4 103 610;
Arylsulfid-Derivate, wie z.B. beschrieben in DE-A-4 031 705;
Phenolderivate, wie z.B. beschrieben in EP-A-0 258 651; Phosphoniumverbindungen und fluorierte Phosphoniumverbindungen, wie z.B. beschrieben in US-A-5 021 473 und US-A-5 147 748;
Calix(n)arene, wie z.B. beschrieben in EP-A-0 385 580;
Benzimidazolone, wie z.B. beschrieben in EP-A-0 347 695;
Ringförmig verknüpfte Oligosaccharide, wie z.B. beschrieben in DE-A-4 418 842; Polyestersalze, wie z.B. beschrieben in DE-A-4 332 170;
Cyclooligosaccharid-Verbindungen, wie z.B. beschrieben in DE-A-197 11 260; Inter-Polyelektrolyt-Komplexe, wie z.B. beschrieben in DE-A-197 32 995.
Weiterhin geeignet, insbesondere für Flüssigtoner, sind oberflächenaktive, ionische Verbindungen und sogenannte Metallseifen.

Besonders geeignet sind alkylierte Arylsulfonate, wie Bariumpetronate, Calciumpetronate, Bariumdinonylnaphthalensulfonate (basisch und neutral), Calciumdinonylsulfonat oder Dodecylbenzolsulfonsäure-Na-salz und Polyisobutylensuccinimide (Chevrons Oloa 1200).
Weiterhin geeignet sind Soyalecithin und N-Vinylpyrrolidon-Polymere.
Weiterhin geeignet sind Natriumsalze von phosphatierten Mono- und Diglyceriden mit gesättigten und ungesättigten Substituenten, AB- Diblockcopolymere von A:
Polymere von 2-(N;N)di-methylaminoethyl-methacrylat quartemisiert mit Methyl-p-toluolsulfonat, und B: Poly-2-ethylhexylmethacrylat.
Weiterhin geeignet, insbesondere in Flüssigtonern, sind di- und trivalente Carboxylate, insbesondere Aluminium-tristearat, Bariumstearat, Chromstearat, Magnesiumoktoat, Calziumstearat, Eisennaphthalat und Zinknaphthalat.
Weiterhin sind geeignet chelatisierende Ladungssteuermittel (EP 0 636 945 A1), metallische (ionische) Verbindungen (EP 0 778 501 A1), Phosphat-Metallsatze, wie in JA 9 (1997)-106107 beschrieben. Weiterhin geeignet sind Azine der folgenden Color-Index-Nummern: C.I. Solvent Black 5, 5:1, 5:2, 7, 31 und 50; C.I. Pigment Black 1, C.I. Basic Red 2 und C.I. Basic Black 1 und 2.

Die erfindungsgemäß verwendeten Struktursilikate werden einzeln oder in Kombination miteinander oder mit weiteren, vorstehend genannten Ladungssteuermitteln, in einer Konzentration von 0,01 bis 50 Gew.-%, vorzugsweise von 0,05 bis 20 Gew.-%, besonders bevorzugt von 0,1 bis 5,0 Gew.-%, bezogen auf die Gesamtmischung, in das Bindemittel des jeweiligen Toners, Entwicklers, Lacks, Pulverlacks, Elektretmaterials oder des elektrostatisch zu trennenden Polymers homogen, beispielsweise durch Extrudieren oder Einkneten, Perlmahlen oder mit Ultraturrax (Schnellrührer) eingearbeitet. Dabei können die erfindungsgemäß eingesetzten Verbindungen als getrocknete und gemahlene Pulver, Dispersionen oder Lösungen, Preßkuchen, Masterbatches, Präparationen, angeteigte Pasten, als auf geeignete Träger, wie z.B. Kieselgel bzw. mit solchen Trägem vermischt, TiO₂, Al₂O₃, Ruß, aus wäßriger oder nicht-wäßriger Lösung aufgezogene Verbindungen oder in sonstiger Form zugegeben werden. Ebenso können die erfindungsgemäß verwendeten Verbindungen grundsätzlich auch schon bei der Herstellung der jeweiligen Bindemittel zugegeben werden, d.h. im Verlauf von deren Polymerisation, Polyaddition oder Polykondensation.

Um elektrophotographische Bunttoner herzustellen, werden Farbmittel wie organische Buntpigmente, anorganische Pigmente oder Farbstoffe, zugesetzt.
Die organischen Buntpigmente können aus der Gruppe der Azopigmente oder polycyclischen Pigmente oder Mischkristalle (solid solutions) solcher Pigmente sein.

Bevorzugte Blau- und/oder Grünpigmente sind Kupferphthalocyanine, wie C.I. Pigment Blue 15, 15:1, 15:2, 15:3, 15:4, 15:6, P. Blue 16(metallfreies Phthalocyanin), oder Phthalocyanine mit Aluminium, Nickel, Eisen oder Vanadium als Zentralatom, weiterhin Triarylcarboniumpigmente, wie Pigment Blue 1,2,9,10, 14, 62, 68, Pigment Green 1, 4, 7, 45; Orange-Pigmente, wie z.B. P.O. 5, 62, 36, 34, 13, 43, 71; Gelbpigmente, wie z.B. P.Y. 12, 13, 17, 83, 93, 122, 155, 180, 174, 185, 97, Rot-Pigmente, wie z.B. P.R. 48, 57, 122, 146, 149, 184, 186, 202, 207, 209, 254, 255, 269, 270, 272, Violett-Pigmente wie P.V. 1, 19, Ruß, Eisen/Mangan-Oxide; weiterhin Mischkristalle aus C.I. Pigment Violett 19 und C.I. Pigment Red 122.
Die Mischungen können in Form der Pulver, durch Mischen von Preßkuchen, sprühgetrockneten Preßkuchen, Masterbatches sowie durch Dispergieren (Extrusion, Kneten, Walzenstuhlverfahren, Perlmühlen, Ultraturrax) in Gegenwart eines Trägermaterials in fester oder flüssiger Form (Tinten auf wäßriger und nicht-wäßriger Basis) sowie durch Flushen in Gegenwart eines Trägermaterials hergestellt werden.

Wird das Farbmittel mit hohen Wasser- oder Lösemittelanteilen eingesetzt (> 5 %), so kann das Mischen auch in Gegenwart erhöhter Temperaturen und durch Vakuum unterstützt ablaufen. Der Flushvorgang kann in Gegenwart oder Abwesenheit von organischen Lösemitteln und von Wachsen ablaufen.

Insbesondere zur Steigerung der Brillanz, aber auch zur Nuancierung des Farbtones bieten sich Mischungen mit organischen Farbstoffen an. Als solche sind bevorzugt zu nennen:
wasserlösliche Farbstoffe, wie z.B. Direct, Reactive und Acid Dyes, sowie lösemittellösliche Farbstoffe, wie z.B. Solvent Dyes, Disperse Dyes und Vat Dyes. Als Beispiele seien genannt: C.I. Reactive Yellow 37, Acid Yellow 23, Reactive Red 23, 180, Acid Red 52, Reactive Blue 19, 21, Acid Blue 9, Direct Blue 199, Solvent Yellow 14, 16, 25, 56, 62, 64, 79, 81, 82, 83, 83:1, 93, 98, 133, 162, 174, Solvent Red 8, 19, 24, 49, 89, 90, 91, 92. 109, 118, 119, 122, 124, 127, 135, 160, 195, 212, 215, Solvent Blue 44, 45, Solvent Orange 41, 60, 63, Disperse Yellow 64, Vat Red 41, Solvent Black 45, 27.
Auch können Farbstoffe und Pigmente mit fluoreszierenden Eigenschaften, wie ^{®}Luminole (Riedel-de Haen) eingesetzt werden, beispielsweise um fälschungssichere Toner herzustellen.

Anorganische Pigmente, wie beispielsweise TiO₂ oder BaSO₄, dienen in Mischungen zur Aufhellung. Weiterhin sind Mischungen mit Effekt-Pigmenten, wie beispielsweise Perlglanz-Pigmenten, Fe₂O₃-Pigmenten (^{®}Paliochrome) sowie Pigmenten auf Basis cholesterischer Polymere, die in Abhängigkeit vom Beobachtungswinkel unterschiedliche Farbeindrücke ergeben, geeignet.

Gegenstand der vorliegenden Erfindung ist auch ein elektrophotographischer Toner, Pulver oder Pulverlack, enthaltend 30 bis 99,99 Gew.-%, vorzugsweise 40 bis 99,5 Gew.%, eines üblichen Bindemittels, beispielsweise ein Styrol-, Styrolacrylat-, Styrolbutadien-, Acrylat-, Urethan-, Acryl-, Polyester- oder Epoxidharz oder eine Kombination der letzten beiden, 0,01 bis 50 Gew.-%, vorzugsweise 0,05 bis 20 Gew.-%, besonders bevorzugt 0,1 bis 5 Gew.-%, mindestens eines Salzes ionischer Struktursilikate und gegebenenfalls 0,001 bis 50 Gew.-%, vorzugsweise 0,05 bis 20 Gew.-%, eines Farbmittels, jeweils bezogen auf das Gesamtgewicht des elektrophotographischen Toners, Pulvers oder Pulverlack.

Ferner können die erfindungsgemäß beschriebenen Verbindungen auf "Free-Flow Agents" als zusätzliches Ladungssteuerelement in suspendierter Form oder in trockener Abmischung aufgebracht werden. Die erfindungsgemäß beschriebenen Verbindungen können auch für ein "Carrier-Coating" eingesetzt werden.

In den nachfolgenden Beispielen bedeuten Teile Gewichtsteile und Prozent Gewichtsprozent.

### Herstellungsbeispiel 1

10 g Bentonit (pH 7-12) werden in 300 ml deionisiertem Wasser 16 Stunden bei 20°C mittels Rühren dispergiert. Dann wird die Suspension mittels verdünnter Schwefelsäure auf einen pH-Wert zwischen 1,5 und 8 eingestellt und danach 5,3 g einer 77 %igen wässrigen Distearyldimethylammoniumchlorid-Lösung (DSDMAC) zur Bentonit-Suspension zugegeben. Die Reaktionsmischung wird anschließend bei 60°C 4 Stunden nachgerührt, abgesaugt, mehrmals mit deionisiertem Wasser nachgewaschen und anschließend bei 60°C im Vakuum getrocknet. Charakterisierung:

### Weißes bis hellgraues Pulver

| | |
|---|---|
| DTA: | keine Zersetzung bis 190°C |
| pH: | 8,4 |
| Leitfähigkeit: | 0,062 mS/cm |
| Restfeuchte: | 1,4 % (Karl-Fischer-Titration) |
| tan δ (1 kHz): | 0,78 |
| Ωcm: | 5·10⁸ |
| Kristallinität: | > 70 % (Röntgenbeugung); zahlreiche Reflexionspeaks zwischen 2 Theta 5 und 55° (Hauptpeaks: 3,5°; 6,6°; 19,8°; 23,7°; 24,4°; 27,7°; 35,0°; 38,3°; 54,0°). |
| Löslichkeiten: | unlöslich in Wasser, Ethanol, Aceton, n-Hexan (< 10 mg/l). |

### Herstellungsbeispiel 2

10 g eines Magnesiumhydrosilikats (Optigel SH, "Hectorit") werden in 400 ml deionisiertem Wasser 2 h bei Raumtemperatur dispergiert.
Dann werden 6,0 g eines 80 %igen wässrigen Distearylmethylbenzyl-/ Distearyldimethyl-ammoniumchlorid-Gemisches (DSMB/DSDMAC) zugegeben und die Reaktionsmischung bei 80-100°C 30 min gerührt. Der Niederschlag wird abgesaugt, mehrmals mit deionisiertem Wasser gewaschen und bei 60°C im Vakuum getrocknet.

### Herstellungsbeispiele 3 bis 28

| Nr. | Herstellung nach Bsp. | verwendetes Struktursilikat | organisches Kation |
|---|---|---|---|
| 3 | 2 | Hectorit | fluoriertes Quat |
| 4 | 1 | Montmorillonit | fluoriertes Quat |
| 5 | 1 | saures Bentonit | DSMB/DSDMAC |
| 6 | 1 | saures Bentonit | fluoriertes Quat |
| 7 | 1 | Magnesiumhydrosilikat | protoniertes prim. Amin (C₁₆/₁₈) |
| 8 | 1 | Magnesiumhydrosilikat | protoniertes primäres Amin (C₈) |
| 9 | 1 | alkalisches Bentonit | Cetyltrimethylammonium |
| 10 | 1 | alkalisches Bentonit | Cocosalkyldimethylbenzyl-ammonium |
| 11 | 1 | alkalisches Bentonit | Didecyldimethylammonium |
| 12 | 1 | alkalisches Bentonit | Dioctyldimethylammonium |
| 13 | 1 | alkalisches Bentonit | fluoriertes Quat |
| 14 | 1 | alkalisches Bentonit | Triphenylmethankation |
| 15 | 1 | alkalisches Bentonit | Diallyldimethylammonium |
| 16 | 1 | alkalisches Bentonit | Tetrapropylammonium |
| 17 | 1 | alkalisches Bentonit | (R)₃N-CH₃ R= (CH₂)₂O-CO(CH₂)₁₁₋₂₁CH₃ |
| 18 | 1 | alkalisches Bentonit | C₁₂/C₁₄-Alkyldimethylbetain |
| 19 | 1 | alkalisches Bentonit | Sulfinato-Sulfobetain |
| 20 | 1 | alkalisches Bentonit | Trimethyltriaza-cyclononanium |
| 21 | 1 | alkalisches Bentonit | Zn-Salicylat 1:1-Komplex |
| 22 | 1 | alkalisches Bentonit | Cetylpyridinium |
| 23 | 1 | wässriges Bentonit | Distearyldimethylammonium |
| 24 | 1 | Kaolinit | Distearyldimethylammonium |
| 25 | 1 | Magnesiumhydrosilikat | Distearyldimethylammonium |
| 26 | 1 | alkalisches Bentonit | Methylenblau |
| 27 | 1 | dto. | 1-Methyl-1-stearylamidoethyl-2-stearyl-imidazolinium |
| 28 | 1 | dto. | Methyl-bis(stearylamidoethyl)-poly(ethylenoxid)ammonium |

fluor. Quat: R-CF=CH-CH₂-N⁺Et₂Me
R = C₅F₁₁ bis C₁₁F₂₃

### Charakterisierung Herstellungsbeispiel 11:

weißes bis hellgraues Pulver
DTA: keine Zersetzung bis 200 °C
pH: 8,7
Leitfähigkeit: 0,09 mS/cm
Restfeuchte: 1,0 % (Karl Fischer-Titration)
SCD: U = -150 mV(10 ml 0,5 % Suspension); Titration bis
   U = 0 mV mit 0,1 ml 10⁻³m Polydadmac-Lösung,
tan δ (1 kHz): 2,7
Ω cm: 6·10⁷
Kristallinität: > 70 % (Röntgenbeugung); zahlreiche Reflexionspeaks zwischen 2 Theta 5 und 55 ° (Hauptpeaks: 4,9°; 9,7°; 19,8°; 23,6°; 24,9°, 29,9°: 35,0°; 45,3°; 54,0°)
Teilchengrößenverteilung: d₅₀ = 26 µm, d₉₅ = 213 µm (Laserlichtbeugung)
BET: 23,4 m²/g
Löslichkeiten: unlöslich in Wasser, Ethanol, Aceton, n-Hexan (< 10 mg/l).

### Charakterisierung Herstellungsbeispiel 13:

### weißes bis hellgraues Pulver

| | |
|---|---|
| DTA: | keine Zersetzung bis 250°C |
| pH: | 5,0 |
| Leitfähigkeit: | 0,20 mS/cm |
| Restfeuchte: | 1,6 % (Karl Fischer-Titration) |
| SCD: | U = -210 mV (10 ml 0,5 % Suspension); Titration bis |
| | U = 0 mit 0,22 ml 10⁻³m Polydadmac-Lösung, |
| tan δ (1 kHz): | 1,3 |
| Ω cm: | 6·10⁸ |
| Kristallinität: | > 70 % (Röntgenbeugung); zahlreiche Reflexionspeaks zwischen 2 Theta 5 und 55° (Hauptpeaks: 6,0°; 18,3°; 19,8°; 24,5°; 30,7°; 34,9°; 38,3°, 43,4°, 54,0°) |
| Teilchengrößenverteilung: | d₅₀ =90 µm, d₉₅ = 390 µm (Laserlichtbeugung) |
| BET: | 17,8 m²/g |
| Löslichkeiten: | unlöslich in Wasser, Ethanol, Aceton, n-Hexan (< 10 mg/l). |

### Anwendungsbeispiele

### Anwendungsbeispiel 1

1 Teil der Verbindung aus Herstellungsbeispiel 1 wird mittels eines Kneters innerhalb von 30 Min. in 99 Teile eines Tonerbindemittels (Styrol-Acrylat-Copolymer 60:40 ^{®}Dialec S 309) homogen eingearbeitet. Anschließend wird auf einer Labor-Universalmühle gemahlen und dann auf einem Zentrifugalsichter klassifiziert. Die gewünschte Teilchenfraktion (4 bis 25 µm) wird mit einem Carrier aktiviert, der aus mit Styrol-Methacrylat-Copolymer (90:10) beschichteten Magnetit-Teilchen der Größe 50 bis 200 µm besteht.

### Anwendungsbeispiel 2

Es wird wie in Anwendungsbeispiel 1 verfahren, wobei, anstelle des Styrol-Acrylat-Copolymers ein Polyesterharz auf Bisphenol-A-Basis (^{®}Almacryl T 500) und als Carrier mit Silikon beschichtete Ferrit-Teilchen der Größe 50 - 200 µm verwendet werden.

Die Messung erfolgt an einem üblichen q/m-Meßstand. Durch Verwendung eines Siebes mit einer Maschenweite von 45 µm wird sichergestellt, daß bei den Tonerausblasungen kein Carrier mitgerissen wird. Die Messungen erfolgen bei ca. 50 %iger relativer Luftfeuchte. In Abhängigkeit von der Aktivierdauer werden folgende q/m-Werte [µC/g] gemessen:

| Aktivierdauer | Anwendungsbeispiel | |
|---|---|---|
| | 2 | 1 |
| | Aufladung q/m [µC/g] | |
| 5 Min. | -22 | -20 |
| 10 Min. | -18 | -21 |
| 30 Min. | -13 | -18 |
| 2 Std. | -11 | -9 |

### Anwendungsbeispiele 3 bis 37:

Es wird wie in Anwendungsbeispiel 1 oder 2 verfahren, wobei anstelle der Verbindung aus Herstellungsbeispiel 1 die unten aufgeführten Verbindungen eingesetzt werden.

| Bsp. | eingesetzte | eingearbeitet gemäß | q/m [µC/g] | | | | |
|---|---|---|---|---|---|---|---|
| | Verbindung | Anwendungsbsp. | 5 Min. | 10 Min. | 30 Min. | 2 Std. | 24 Std. |
| 3 | Magnesiumhydrosilikat (®Optigel SH) | 1 | - 6 | - 6 | - 8 | - 8 | |
| 4 | saures Bentonit | 2 | - 9 | - 8 | - 5 | - 4 | - 3 |
| 5 | saures Bentonit | 1 | - 4 | -7 | -10 | -9 | -12 |
| 6 | Montmorillonit K 10 | 2 | - 8 | - 6 | - 4 | -1 | |
| 7 | Montmorillonit K 10 | 1 | - 4 | - 6 | - 7 | - 6 | - 6 |
| 8 | Optigel WM | 1 | -1 | - 2 | - 2 | - 3 | |
| 9 | Kaolinit | 1 | ± 0 | -1 | - 1 | - 2 | |
| 10 | alkalisches Bentonit | 1 | - 7 | -10 | - 10 | -19 | |
| 11 | Herstellungsbeispiel 2 | 1 | + 4 | +7 | +12 | + 18 | + 21 |
| 12 | Herstellungsbeispiel 3 | 1 | + 2 | +7 | + 13 | + 14 | +16 |
| 13 | Herstellungsbeispiel 3 | 2 | + 3 | +4 | + 3 | + 2 | + 1 |
| 14 | Herstellungsbeispiel 4 | 1 | + 1 | + 3 | + 8 | + 9 | + 10 |
| 15 | Herstellungsbeispiel 5 | 1 | + 1 | + 5 | +10 | + 17 | + 21 |
| 16 | Herstellungsbeispiel 6 | 1 | + 2 | + 6 | + 10 | + 12 | +10 |
| 17 | Hectorit + DSDMAC | 1 | + 3 | + 6 | + 10 | + 15 | + 19 |
| 18 | Herstellungsbeispiel 9 | 1 | -13 | -14 | -16 | -13 | |
| 19 | Herstellungsbeispiel 10 | 1 | -15 | - 16 | -16 | -13 | |
| 20 | Herstellungsbeispiel 11 | 1 | -18 | - 20 | -18 | -14 | |
| 21 | Herstellungsbeispiel 12 | 1 | -13 | -14 | -14 | -12 | |
| 22 | Herstellungsbeispiel 13 | 1 | - 20 | - 21 | -19 | -15 | |
| 23 | Herstellungsbeispiel 14 | 1 | - 11 | -13 | -13 | - 10 | |
| 24 | Herstellungsbeispiel 15 | 1 | - 5 | - 6 | - 6 | - 4 | |
| 25 | Herstellungsbeispiel 16 | 1 | -17 | -16 | - 14 | - 9 | |
| 26 | Herstellungsbeispiel 17 | 1 | - 17 | - 18 | - 16 | - 10 | |
| 27 | Herstellungsbeispiel 18 | 1 | - 16 | - 16 | - 17 | - 13 | |
| 28 | Herstellungsbeispiel 19 | 1 | -13 | -16 | -19 | -16 | |
| 29 | Herstellungsbeispiel 20 | 1 | - 7 | - 8 | - 9 | - 8 | |
| 30 | Herstellungsbeispiel 21 | 1 | -14 | -16 | -16 | -14 | |
| 31 | Herstellungsbeispiel 22 | 1 | -13 | -17 | -18 | -15 | |
| 32 | Herstellungsbeispiel 23 | 1 | - 2 | - 2 | - 2 | - 2 | |
| 33 | Herstellungsbeispiel 24 | 1 | - 1 | ± 0 | ± 0 | + 2 | |
| 34 | Herstellungsbeispiel 25 | 1 | + 3 | + 6 | +10 | + 15 | + 20 |
| 35 | Herstellungsbeispiel 26 | 1 | -13 | -14 | -14 | -13 | |
| 36 | Herstellungsbeispiel 27 | 1 | - 15 | - 16 | - 17 | -17 | |
| 37 | Herstellungsbeispiel 28 | 1 | -16 | -18 | -19 | -19 | |

### Anwendungsbeispiele 38bis 40

Es wird wie in Anwendungsbeispiel 1 verfahren, wobei anstelle von 1 Teil jeweils 0,5, 2 oder 3 Teile der Verbidung aus Herstellungsbeispiel 1 eingesetzt werden.

### Anwendungsbeispiele 41 und 42

Es wird wie in Anwendungsbeispiel 2 verfahren, wobei anstelle von 1 Teil 2 bzw. 3 Teile der Verbindung aus Herstellungsbeispiel 1 eingesetzt werden.

| Bsp. | | q/m [µC/g] | | | | |
|---|---|---|---|---|---|---|
| Nr. | Teile | 5 Min. | 10 Min. | 30 Min. | 2 Std. | 24 Std. |
| 38 | 0,5 | -17 | -19 | -15 | -6 | |
| 39 | 2 | -32 | -30 | -21 | -6 | |
| 40 | 3 | - 36 | - 31 | - 23 | - 14 | |
| 41 | 2 | -28 | -25 | -23 | -21 | -19 |
| 42 | 3 | -32 | -26 | -24 | -23 | -21 |

### Anwendungsbeispiele 43 und 44

Es wird wie in Anwendungsbeispiel 1 verfahren, wobei zu dem einen Teil der Verbindung aus Herstellungsbeispiel 1 noch 1 bzw. 5 Teile eines Farbmittels mit elektrostatisch positivem Eigeneffekt (C.I. Solvent Blue 125, siehe Vergleichsbeispiel A) eingearbeitet werden.

| | | q/m [µC/g] | | | | |
|---|---|---|---|---|---|---|
| Nr. | Teile Farbmittel | 5 Min. | 10 Min. | 30 Min. | 2 Std. | 24 Std. |
| 43 | 1 | -17 | -15 | -11 | -7 | -5 |
| 44 | 5 | -5 | -4 | -3 | -4 | -3 |

### Anwendungsbeispiele 45 bis 53

Es wird wie in den Anwendungsbeispielen 1, 38 und 39 verfahren, wobei zusätzlich noch 5 Teile eines organischen Pigments (Ruß®Mogul L, Cabot;®Toner Magenta E02, Clariant (C.I. P. Red 122); ®Toner Yellow HG, Clariant (C.I. P. Yellow 180)) eingearbeitet werden.

| | Teile Verbindung | organisches | q/m [µC/g] | | | | |
|---|---|---|---|---|---|---|---|
| Nr. | Herstellungsbsp. | 1 pigment | 5 Min. | 10 Min. | 30 Min. | 2 Std. | 24 Std. |
| 45 | 0,5 | Toner Magenta EO2 | - 16 | - 14 | -10 | - 8 | - 5 |
| 46 | 1 | Toner Magenta EO2 | - 21 | - 17 | -14 | - 10 | - 3 |
| 47 | 2 | Toner Magenta EO2 | - 22 | - 21 | - 16 | - 7 | - 5 |
| 48 | 0,5 | Toner Yellow HG | - 21 | - 21 | - 19 | - 13 | - 8 |
| 49 | 1 | Toner Yellow HG | - 24 | - 24 | - 21 | - 11 | - 6 |
| 50 | 2 | Toner Yellow HG | - 29 | - 26 | - 22 | - 13 | - 8 |
| 51 | 0,5 | Ruß | - 15 | - 15 | - 12 | -8 | -6 |
| 52 | 1 | Ruß | -20 | - 18 | -15 | -13 | -9 |
| 53 | 2 | Ruß | - 22 | - 20 | - 16 | - 12 | - 8 |

### Vergleichsbeispiel A:

Es wird wie in Anwendungsbeispiel 43 verfahren, wobei 1 Teil C.I. Solvent Blue 125, aber kein erfindungsgemäßes Ladungssteuermittel eingearbeitet wird.

| Aktivierdauer | Aufladung q/m [µC/g] |
|---|---|
| 5 Min | ±0 |
| 10 min | +1 |
| 30 Min | +3 |
| 120 Min | +10 |
| 24 Std. | +29 |

Der ausgeprägte positive triboelektrische Eigeneffekt des blauen Farbmittels ist deutlich erkennbar.

### Anwendungsbeispiel 54

1 Teil der Verbindung aus dem Anwendungsbeispiel 1 wurde in 99 Teile eines Pulverlackbindemittels (^{®}Crylcoat 430) homogen eingearbeitet, wie bei den oben erwähnten Anwendungsbeispielen beschrieben. Die Triboversprühung der Pulver(lacke) wurde mit einem Sprühgerät ^{®}TriboStar der Firma Intec (Dortmund), mit einem Normsprührohr und einer Steminnenstange bei maximalem Pulverdurchsatz mit einem Sprühdruck von 3 und 5 bar durchgeführt. Die aus der elektrostatischen Ladung von Pulverlack oder Pulver sich ergebende Stromstärke wurde in µA angezeigt. Die Abscheidequote wurde anschließend in % durch eine Differenzwiegung aus versprühtem und abgeschiedenem Pulverlack bestimmt.

| Druck [bar] | Strom [µA] | Abscheidequote [%] |
|---|---|---|
| 3 | 3,9 | 40 |
| 5 | 5,3 | 48 |

## Patentansprüche

1. Verwendung von salzartigen Struktursilikaten, worin ein niedermolekülares organisches Kation enthalten ist, welches ein substituiertes Ammonium-, Phosphonium-, Thionium-, Triphenylcarbonium-ion oder ein kationisher Metallkomplex ist und das Anion ein insel-, Ring-, Gruppen-, Ketten-, Bänder-, Schicht-oder Gerüstsilikat oder eine Kombination davon ist, als Ladungssteuermittel in elektrophotographischen Tonern und Entwicklern, in Putvenacken, Elektretmateriallien und in elektrostatischen Trennverfahren.

2. Verwendung nach Anspruch 1, **dadurch gekennzeichnet, dass** das Silikat ein Anion aus der Gruppe Montmorillonit, Bentonit, Hectorit, Kaolinit, Serpentin, Talk, Pyrophyllit, Glimmer, Phlogopit, Biotit, Muscovit, Paragonit, Vermiculit, Beldellit Xantophyllit, Margarit, Feldspat, Zeolith, Wollastonit Aktinolith, Amosit, Krokydolith, Sillimanit, Nontronit, Smectit, Sepiolith, Saponit, Faujasith, Permutit und Sasil ist.

3. Verwendung nach Anspruch 1, **dadurch gekennzeichnet, dass** das Ammoniumion eine der Formeln (a) - (j) hat, 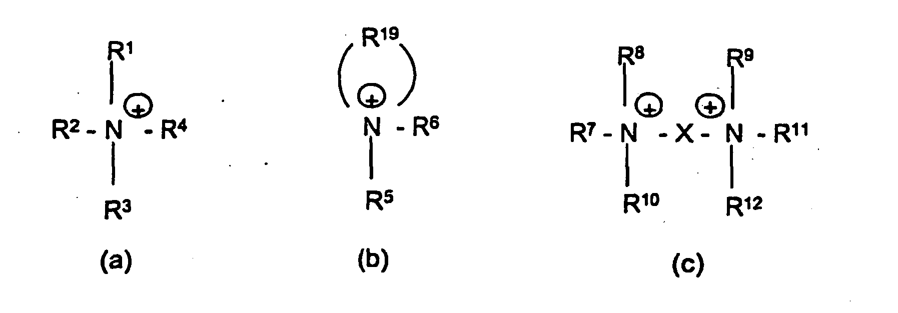 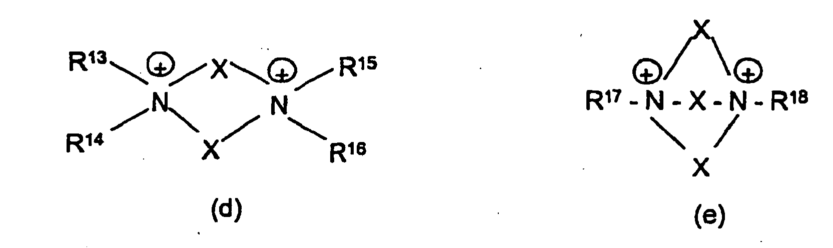 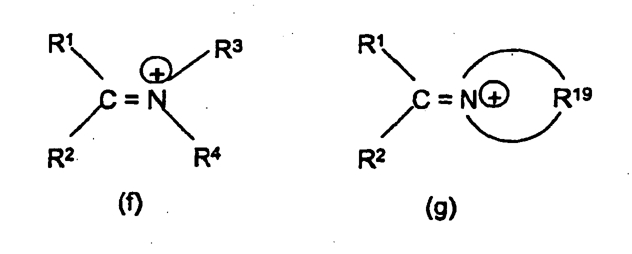 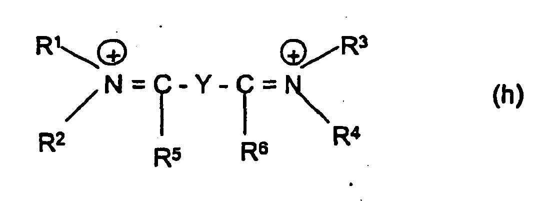 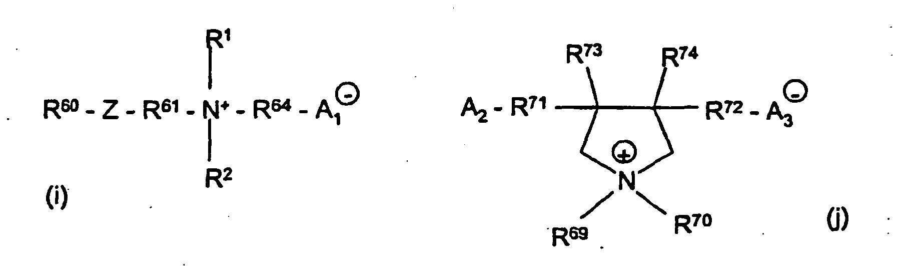 worin
R¹ bis R¹⁸ gleich oder verschieden sind und für Wasserstoff, CN, (CH₂)₁₋₈-CN, Halogen, verzweigtes oder unverzweigtes C₁-C₃₂-Alkyl, ein- oder mehrfach ungesättigtes C₂-C₃₂-Alkenyl, C₁-C₂₂-Alkoxy, C₁-C₂₂-Hydroxyalkyl, C₁-C₂₂-Halogenalkyl, C₂-C₂₂-Halogenalkenyl, C₁-C₂₂-Aminoalkyl, (C₁-C₁₂)-Trialkylammonium-(C₁-C₂₂)-alkyl;
(C₁-C₂₂)-Alkylen-(C=O)O-(C₁-C₃₂)alkyl, (C₁-C₂₂)-Alkylen-(C=O)O-aryl, (C₁-C₂₂)-Alkylen-(C=O)NH-(C₁-C₃₂)alkyl, (C₁-C₂₂)-Alkylen-(C=O)NH-aryl,
(C₁-C₂₂)Alkylen-O(CO)-(C₁-C₃₂)alkyl, (C₁-C₂₂)-Alkylen-O(CO)-aryl, (C₁-C₂₂)Alkylen-NH(C=O)-(C₁-C₃₂)alkyl, (C₁-C₂₂)-Alkylen-NHCO-aryl; wobei in die Säureester oder Säureamidbindungen 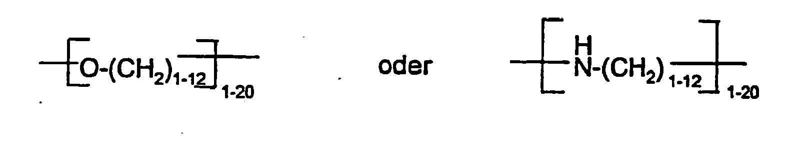 eingeschoben sein kann;
[(C₁-C₁₂)-alkylen-O-]₁₋₁₀₀-H; Aryl, (C₁-C₁₈)-Alkylen-aryl; -(O-SiR'₂)₁₋₃₂-O-SiR'₃, wobei R' die Bedeutung C₁-C₁₂-Alkyl, Phenyl, Benzyl oder C₁-C₁₂-Alkoxy hat; Heterocyclus, C₁-C₁₈-Alkylen-heterocyclus, wobei die Aryl- und Heterocyclusreste an Kohlenstoff oder Heteroatomen einfach oder mehrfach durch C₁-C₁₂Alkyl, C₁-C₄-Alkenyl, C₁-C₄-Alkoxy, Hydroxy-(C₁-C₄)alkyl, Amino-(C₁-C₄)alkyl, C₁-C₄-Alkylimino, Carboxy, Hydroxy, Amino, Nitro, Cyano, Halogen, C₁-C₁₂-Acyl, C₁-C₄-Halogenalkyl, C₁-C₄-Alkylcarbonyl, C₁-C₄-Alkylcarbonyloxy, C₁-C₄-Alkoxycarbonyl, C₁-C₄-Alkylaminocarbonyl, C₁-C₄-Alkylcarbonylimino, C₆-C₁₀-Arylcarbonyl, Aminocarbonyl, Aminosulfonyl, C₁-C₄-Alkylaminosulfonyl, Phenyl, Naphthyl, Heteroaryl, substituiert sein können;
R¹⁹ für C₄-C₁₁-Alkylen, -(C₂H₄-O-)₁₋₁₇-(CH₂)₁₋₂-, -(C₂H₄-NR-)₁₋₁₇-(CH₂)₁₋₂-, wobei R Wasserstoff oder C₁-C₁₂-Alkyl ist;
X die Bedeutung von Y sowie -CO-CH₂-CO-, 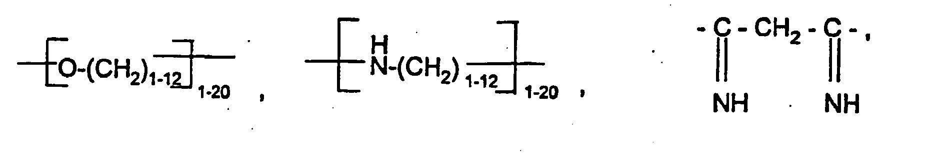 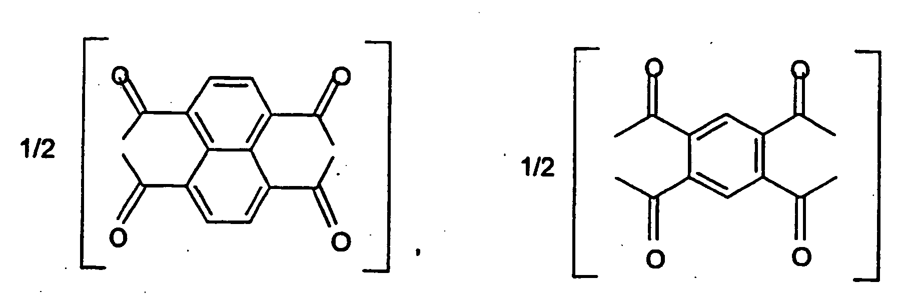 oder 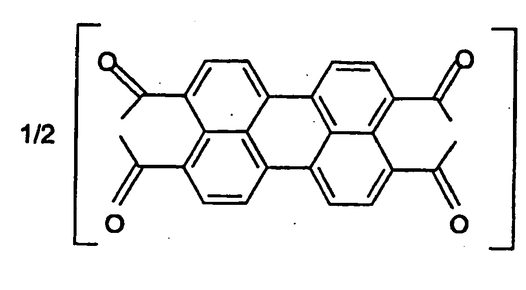 hat;
Y die Bedeutung 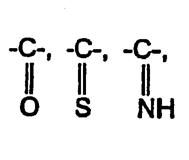 -(CH₂)₁₋₁₈-_{,} 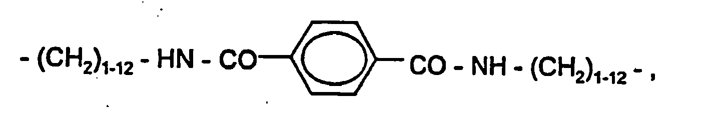 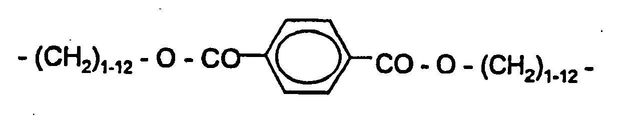 oder o-, p-, m-(C₆-C₁₄)Arylen oder (C₄-C₁₄)Heteroarylen mit 1, 2, 3 oder 4 Heteroatomen aus der Gruppe N, O und/oder S hat;
R⁶⁰ für C₁-C₃₂-Acyl, C₁-C₂₂-Alkyl, C₂-C₂₂-Alkenyl, C₁-C₁₈Alkylen-C₆-C₁₀-aryl, C₁-C₂₂-Alkylen-heterocyclus, C₆-C₁₀-Aryl oder (C₄-C₁₄)-Heteroaryl mit 1, 2, 3 oder 4 Heteroatomen aus der Gruppe N, O und/oder S,
R⁶¹ und R⁶⁴ für -(CH₂)₁₋₁₈-, C₁-C₁₂-Alkylen-C₆-C₁₀-arylen, C₆-C₁₀-Arylen, C₀-C₁₂-Alkylen-heterocyclus,
Z für -NH- oder -O- ;
A₁^{θ} und A₃^{θ} für -COO^{θ}, -SO₃^{θ}, -OSO₃^{θ}, SO₂^{θ}, -COS^{θ} oder -CS₂^{θ};
A₂ für -SO₂Na, -SO₃Na, -SO₂H, SO₃H oder Wasserstoff.
R⁶⁹ und R⁷⁰ unabhängig voneinander für Wasserstoff, C₁-C₃₂-Alkyl, wobei in der Alkylkette eine oder mehrere der Gruppen-NH-CO-, -CO-NH-, -CO-O- oder -O-COenthalten sein kann; C₁-C₁₈-Alkylen-aryl, C₀-C₁₈₋Alkylen-heterocyclus. C₁-C₁₈-Hydroxyalkyl, C₁-C₁₈-Halogenalkyl, Aryl. -(CH₂)₃-SO₃^{θ}, 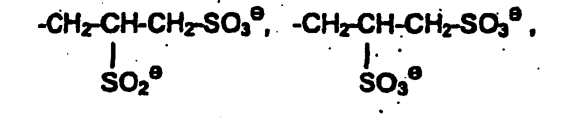 R⁷¹ und R⁷² für -(CH₂)₁₋₁₂- ; und
R⁷³ und R⁷⁴ für Wasserstoff oder C₁-C₂₂-Alkyl stehen.

4. Verwendung nach Anspruch 3, **dadurch gekennzeichnet, daß** R¹ bis R¹⁸ Wasserstoff, CN, CH₂-CN, CF₃, C₁-C₂₂-Alkyl, C₂-C₁₈-Alkenyl, C₁-C₁₈-Alkoxy, C₁-C₁₈-Hydroxy-alkyl, C₁-C₁₈-Halogenalkyl, C₂-C₁₈-Halogenalkenyl, wobei Halogen vorzugsweise F oder Cl bedeutet. C₁-C₁₈-Aminoalkyl, (C₁-C₈)-Trialkylammonium-(C₁C₁₈)-alkyl, (C₁-C₁₈)-Alkylen-O(C=O)-(C₁-C₂₂)alkyl, (C₁-C₁₈)-Alkylen-O(C=O)-phenyl, (C₁-C₁₈)-Alkylen-NHCO-(C₁-C₂₂)alkyl, (C₁-C₁₈)-Alkylen-NHCO-phenyl, (C₁-C₁₈)-Alkylen-(C=O)O-(C₁-C₂₂)alkyl, (C₁-C₁₈)-Alkylen-(C=O)O-phenyl, (C₁-C₁₈)Alkylen-(C=O)NH-(C₁-C₂₂)alkyl, (C₁-C₁₈)-Alkylen-CONH-phenyl, Benzyl, Phenyl, Naphthyl, C₁-C₁₂-Alkylen-heterocyclus;
R¹⁹ C₄-C₅-Alkylen, -(C₂H₄-O)₁₋₈-(CH₂)₁₋₂-, -(C₂H₄-NH)₁₋₉(CH₂)₁₋₂-;
R⁶⁰ C₁-C₁₈-Acyl, C₁-C₁₈-Alkyl, C₂-C₁₈-Alkenyl, C₁-C₁₂-Alkylen-phenyl, C₁-C₁₈-Alkylen-pyridyl, Phenyl, Pyridyl;
R⁶¹ und R⁶⁴ -(CH₂)₁₋₁₂-, C₁-C₈-Alkylen-phenylen, Phenylen; C₁-C₈-Alkylenpyridylen- oder-piperidylen;
R⁷¹ und R⁷² -(CH₂)₁₋₈ und
R⁷³ und R⁷⁴ Wasserstoff oder (C₁-C₁₈)-Alkyl bedeuten.

5. Verwendung nach Anspruch 1, **dadurch gekennzeichnet, dass** das Ammoniumion ein aliphatischer oder aromatischer 5 bis 12-gliedriger Heterocyclus mit 1 bis 4 ringangehörigen N-, O-und/oder S-Atomen, wobei 2 bis 8 Ringe annelliert sein können, ist, vorzugsweise Pyridinium, Pyridazinium, Pyrimidinium, Pyrazinium, Purinium, Tetraazaporphyrinium, Piperidinium, Morpholinium, Tetrazonium, Triazacyclononanium und Tetraaza-cyclododecanium.

6. Verwendung nach Anspruch 1, **dadurch gekennzeichnet dass** der kationische Metalikomplex ein Metallcarboxylat, Metallsalicylat, Metallsulfonat, 1:1-Metall-Azo-komplex oder ein Metall-dithiorarbamat ist, wobei Metall vorzugsweise Al, Mg, Ca, Sr, Ba, TiO, VO, Cr, V, Ti, Zr, Sc, Mn, Fe, Co, Ni, Cu, Zn und ZrO ist und der Metallkomplex gegebenenfalls ein oder mehrere weitere Liganden enthält.

7. Verwendung nach einem oder mehreren der Ansprüche 1 bis 6, dadruch **gekennzeichnet**, daß das organische Kation ein fluoriertes Ammoniumion der Formel (x) ist 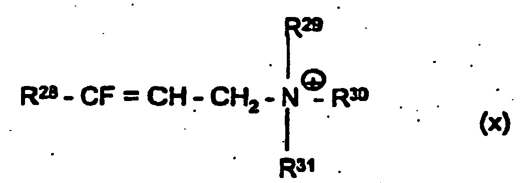 worin.
R²⁸ perfluoriertes Alkyl mit 5 bis 11 C-Atomen,
R²⁸, R³⁰ und R³¹ gleich oder verschieden sind und Alkyl mit 1 bis 5 C-Atomen, vorzugsweise 1 bis 2 C-Atomen, bedeuten.

8. Salzartiges Struktursilikat, worin das Silikat Hectorit, Beidellit Illit, Muskovit, Xantophyllit Margarit, Sepiolith, Saponit, Glimmer, Feldspat, Nontronit Montmorillonit, Smectit, Bentonit, Faujasit, Zeolith A, X oder Y, Permutit, Sasil oder eine Kombination davon: und das Kation ein ion der Formel (x) gemäß Anspruch 7 ist.

9. Verfahren zur Herstellung eines salzartigen Struktursilikats gemäß Anspruch 8, **dadurch gekennzeichnet, dass** man das Silikat und das Salz des Kations der Formel (x) in wäßrigem Medium vereinigt

10. Elektrophotographischer Toner, Pulver oder Pulverlack, enthaltend 30 bis 99,99 Gew.-%, vorzugsweise 40 bis 99,5 Gew.-%, eines Bindemittels, 0,01 bis 50 Gew.-%, vorzugsweise 0,05 bis 20 Gew.-%, mindestens eines Salzartigen Struktursilikate, wie in einem der Ansprüche 1 bis 8 definiert, und gegebenenfalls 0,001 bis 50 Gew.-%, vorzugsweise 0,05 bis 20 Gew.-%, eines Farbmittels, jeweils bezogen auf das Gesamtgewicht des elektrophotographischen Toners, Pulvers oder Pulverlacks.

## Claims

1. The use of a salt-like structured silicate which comprises a low molecular weight organic cation which is a substituted ammonium, phosphonium, thionium or triphenylcarbonium ion or a cationic metal complex and in which the anion is an island, cyclic, group, chain, ribbon, laminar or matrix silicate in which as a charge control agent in electrophotographic toners and developers, in powder coatings, electret materials and in electrostatic separation processes.

2. The use as claimed in claim 1, wherein the silicate is an anion from the group consisting of montmorillonite, bentonite, hectorite, kaolinite, serpentine, talc, pyrophyllite, mica, phlogopite, biotite, muscovite, paragonite, vermiculite, beidellite, xantophyllite, margarite, feldspar, zeolite, wollastonite, actinolite, amosite, crocidolite, sillimanite, nontronite, smectite, sepiolite, saponite, faujasite, permutite and sasil.

3. The use as claimed in claim 1, wherein the ammonium ion has one of the formulae (a) - (j) 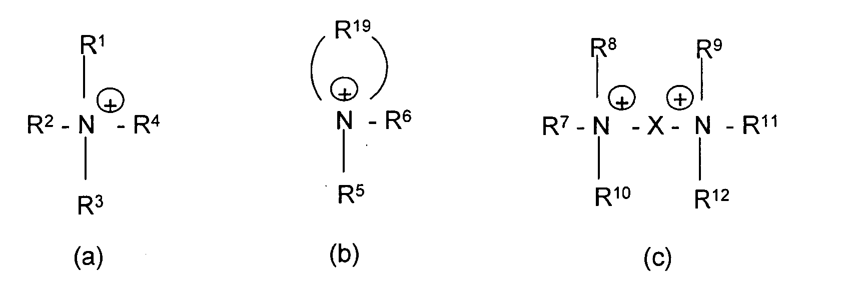 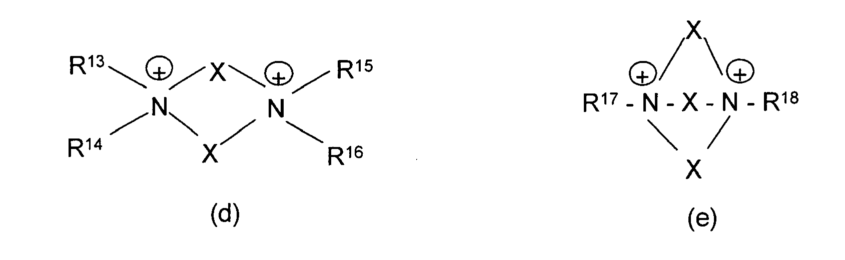 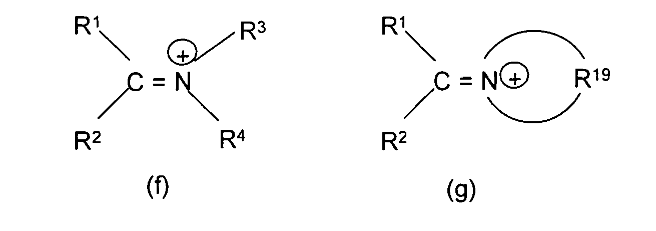 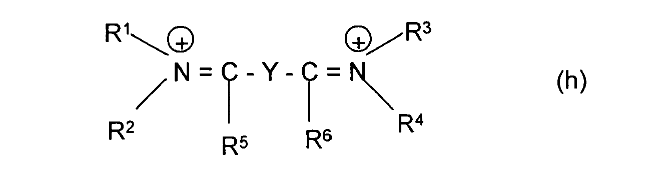 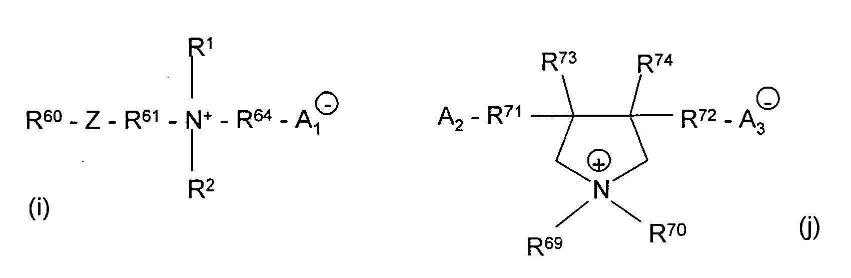 in which
R¹ to R¹⁸ are identical or different and represent hydrogen, CN, (CH₂)₁₋₁₈CN, halogen, branched or unbranched C₁-C₃₂-alkyl, mono- or polyunsaturated C₂-C₃₂-alkenyl, C₁-C₂₂-alkoxy, C₁-C₂₂-hydroxyalkyl, C₁-C₂₂-halogenoalkyl, C₂-C₂₂-halogenoalkenyl, C₁-C₂₂-aminoalkyl, (C₁-C₁₂)-trialkyl-ammonium-(C₁-C₂₂)-alkyl;
(C₁-C₂₂)-alkylene-(C=O)O-(C₁-C₃₂)alkyl, (C₁-C₂₂)-alkylen-(C=O)O-aryl, (C₁-C₂₂)-alkylene-(C=O)NH-(C₁-C₃₂)alkyl, (C₁-C₂₂)-alkylene-(C=O)NH-aryl,
(C₁-C₂₂)-alkylene-O(CO)-(C₁-C₃₂)alkyl, (C₁-C₂₂)-alkylene-O(CO)-aryl, (C₁-C₂₂)-alkylene-NH(C=O)-(C₁-C₃₂)alkyl, (C₁-C₂₂)-alkylene-NHCO-aryl;
wherein 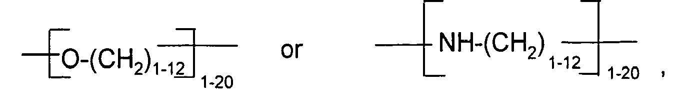 can be inserted into the acid ester or acid amide bonds;
[(C₁-C₁₂)alkylene-O]₁₋₁₀-H; aryl, (C₁-C₁₈)-alkylenearyl; -(O-SiR'₂)₁₋₃₂-O-SiR'₃, in which R' has the meaning C₁-C₁₂-alkyl, phenyl, benzyl or C₁-C₁₂-alkoxy; heterocyclyl, C₁-C₁₈-alkylene-heterocyclyl, wherein the aryl and heterocyclyl radicals can be mono- or polysubstituted on carbon atoms or heteroatoms by C₁-C₁₂-alkyl, C₁-C₄-alkenyl, C₁-C₄-alkoxy, hydroxy-(C₁-C₄)alkyl, amino-(C₁-C₄)alkyl, C₁-C₄-alkylimino, carboxyl, hydroxyl, amino, nitro, cyano, halogen, C₁-C₁₂-acyl, C₁-C₄-halogenoalkyl, C₁-C₄-alkylcarbonyl, C₁-C₄-alkylcarbonyloxy, C₁-C₄-alkoxycarbonyl, C₁-C₄-alkylaminocarbonyl, C₁-C₄-alkylcarbonylimino, C₆-C₁₀-arylcarbonyl, aminocarbonyl, aminosulfonyl, C₁-C₄-alkylaminosulfonyl, phenyl, naphthyl, or heteroaryl,;
R¹⁹ represents C₄-C₁₁-alkylene, -(C₂H₄-O-)₁₋₁₇-(CH₂)₁₋₂-, -(C₂H₄-NR-)₁-₁₇-(CH₂)₁₋₂-, in which R is hydrogen or C₁-C₁₂-alkyl;
X has the meaning of Y and -CO-CH₂-CO-, 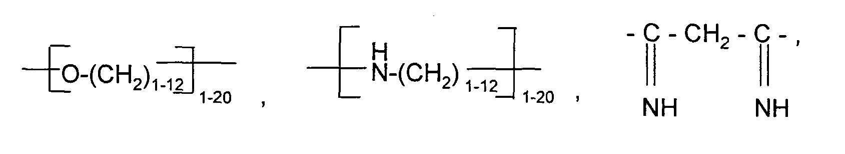 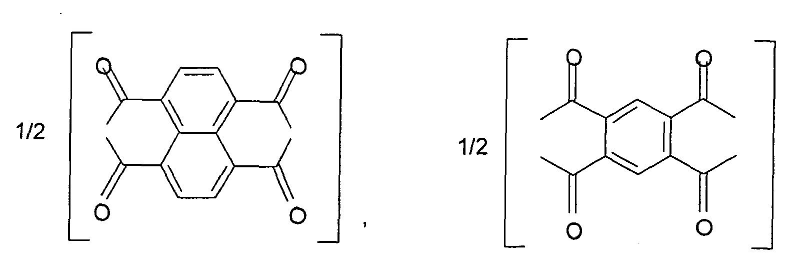 or 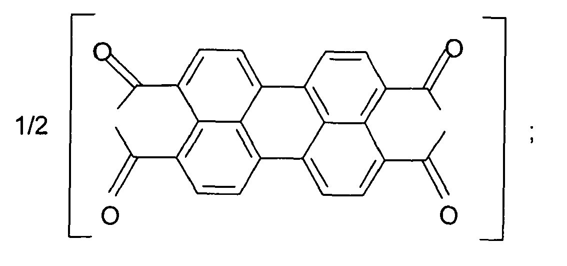 Y has the meaning 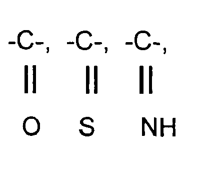 -(CH2)1-18-, 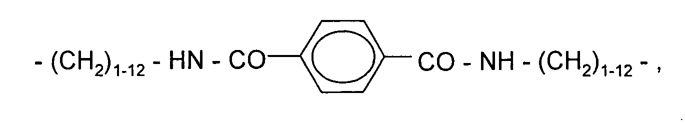 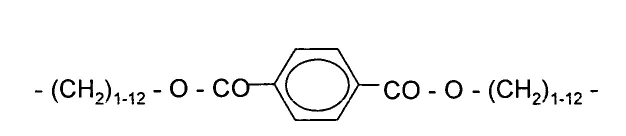 or o-, p-, m-(C₆-C₁₄)-arylene or (C₄-C₁₄)-heteroarylene with 1, 2, 3 or 4 heteroatoms from the group consisting of N, O and/or S;
R⁶⁰ represents C₁-C₃₂-acyl, C₁-C₂₂-alkyl, C₂-C₂₂-alkenyl, C₁-C₁₈-alkylene-C₆-C₁₀-aryl, C₁-C₂₂-alkylene-heterocyclyl, C₆-C₁₀-aryl or (C₄-C₁₄)-heteroaryl with 1, 2, 3 or 4 heteroatoms from the group consisting of N, O and/or S,
R⁶¹ and R⁶⁴ represent -(CH₂)₁₋₁₈-, C₁-C₁₂-alkylene-C₆-C₁₀-arylene, C₆-C₁₀-arylene, C₀-C₁₂-alkylene-heterocyclyl;
Z represents -NH- or -O- ;
R₁^{σ} and A₃^{σ} represent -COO^{σ}, -SO₃^{σ}, -OSO₃^{σ}, -SO₂^{σ}, -COS^{σ} or -CS₂^{σ};
A₂ represents -SO₂Na, -SO₃Na, -SO₂H, -SO₃H or hydrogen;
R⁶⁹ and R⁷⁰ independently of one another represent hydrogen, C₁-C₃₂-alkyl, in which the alkyl chain can contain one or more of the groups -NH-CO-, -CO-NH-, -CO-O- or -O-CO-; C₁-C₁₈-alkylene-aryl, C₀-C₁₈-alkylene-heterocyclyl, C₁-C₁₈-hydroxyalkyl, C₁-C₁₈-halogenoalkyl, aryl, -(CH₂)₃-SO₃^{σ}, 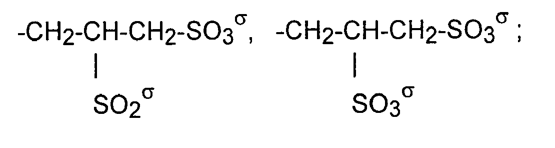 R⁷¹ and R⁷² represent -(CH₂)₁₋₁₂-; and
R⁷³ and R⁷⁴ represent hydrogen or C₁-C₂₂-alkyl.

4. The use as claimed in claim 3, wherein R¹ to R¹⁸ denote hydrogen CN, CH₂-CN, CF₃, C₁-C₂₂-alkyl, C₂-C₁₈-alkenyl, C₁-C₁₈-alkoxy, C₁-C₁₈-hydroxy-alkyl, C₁-C₁₈-halogenoalkyl, C₂-C₁₈-halogenoalkenyl, in which halogen preferably denotes F or Cl, C₁-C₁₈-aminoalkyl, (C₁-C₆)-trialkylammonium-(C₁-C₁₈)-alkyl, (C₁-C₁₈)-alkylene-O(C=O)-(C₁-C₂₂)alkyl, (C₁-C₁₈)-alkylene-O(C=O)-phenyl, (C₁-C₁₈)-alkylene-NHCO-(C₁-C₂₂)alkyl, (C₁-C₁₈)-alkylene-NHCO-phenyl, (C₁-C₁₈)-alkylene-(C=O)O-(C₁-C₂₂)alkyl, (C₁-C₁₈)-alkylene-(C=O)O-phenyl, (C₁-C₁₈)alkylene-(C=O)NH-(C₁-C₂₂)alkyl, (C₁-C₁₈)-alkylene-CONH-phenyl, benzyl, phenyl, naphthyl, C₁-C₁₂-alkylene-heterocyclyl;
R¹⁹ denotes C₄-C₅-alkylene, -(C₂H₄-O)₁₋₉-(CH₂)₁₋₂- or -(C₂H₄-NH)₁₋₉-(CH₂)₁₋₂-;
R⁶⁰ denotes C₁-C₁₈-acyl, C₁-C₁₈-alkyl, C₂-C₁₈-alkenyl, C₁-C₁₂-alkylene-phenyl, C₁-C₁₈-alkylene-pyridyl, phenyl or pyridyl;
R⁶¹ and R⁶⁴ denote -(CH₂)₁₋₁₂-, C₁-C₈-alkylene-phenylene, phenylene or C₁-C₈-alkylenepyridylene or piperidylene;
R⁷¹ and R⁷² denote -(CH₂)₁₋₈ and
R⁷³ and R⁷⁴ denote hydrogen or (C₁-C₁₈)-alkyl.

5. The use as claimed in claim 1, wherein the ammonium ion is an aliphatic or aromatic 5- to 12-membered heterocyclic radical with 1 to 4 N, O and/or S atoms belonging to the rings, it being possible for 2 to 8 rings to be fused, preferably pyridinium, pyridazinium, pyrimidinium, pyrazinium, purinium, tetraazaporphyrinium, piperidinium, morpholinium, tetrazonium, triaza-cyclononanium and tetraaza-cyclododecanium.

6. The use as claimed in claim 1, wherein the cationic metal complex is a metal carboxylate, metal salicylate, metal sulfonate, 1:1 metal-azo complex or a metal dithiocarbamate, in which metal is preferably Al, Mg, Ca, Sr, Ba, TiO, VO, Cr, V, Ti, Zr, Sc, Mn, Fe, Co, Ni, Cu, Zn and ZrO, and the metal complex optionally contains one or more further ligands.

7. The use as claimed in one or more of claims 1 to 6, wherein the organic cation is a fluorinated ammonium ion of the formula (x) 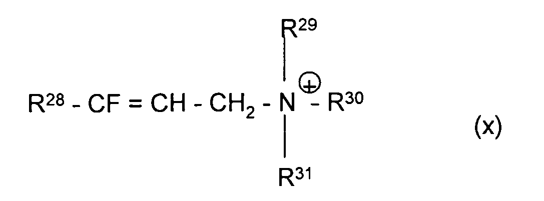 in which
R²⁸ denotes perfluorinated alkyl having 5 to 11 carbon atoms and
R²⁹, R³⁰ and R³¹ are identical or different and denote alkyl having 1 to 5 carbon atoms, preferably 1 or 2 carbon atoms.

8. Salt-like structured silicate, in which the silicate is hectorite, beidellite, illite, muscovite, xantophyllite, margarite, sepiolite, saponite, mica, feldspar, nontronite, montmorillonite, smectite, bentonite, faujasite, zeolite A, X or Y, permutite, sasil or a combination thereof; and the cation is an ion of the formula (x) as claimed in claim 7.

9. A process for the preparation of a salt-like structured silicate as claimed in claim 8, which comprises combining the silicate and the salt of the cation of formula (x) in an aqueous medium.

10. An electrophotographic toner, powder or powder coating comprising 30 to 99.99% by weight, preferably 40 to 99.5% by weight of a binder, 0.01 to 50% by weight, preferably 0.05 to 20% by weight of at least one salt-like structured silicate as defined in any of claims 1 to 8, and optionally 0.001 to 50% by weight, preferably 0.05 to 20% by weight of a coloring agent, in each case based on the total weight of the electrophotographic toner, powder or powder coating.

## Revendications

1. Utilisation de silicates structuraux de type sel qui contiennent un cation organique de faible masse moléculaire, ledit cation étant un ion ammonium, phosphonium, thionium, triphénylcarbonium substitué ou un complexe métallique cationique et l'anion est un nésosilicate, un cyclosilicate, un sorosilicate, un inosilicate à chaîne, un inosilicate à ruban, un phyllosilicate ou un tectosilicate ou une combinaison de ceux-ci, en tant qu'agent de contrôle de charge dans des toners et des révélateurs électrophotographiques, dans des vernis en poudre, des matériels de type électrets et dans des procédés de séparation électrostatiques.

2. Utilisation selon la revendication 1, **caractérisée en ce que** le silicate est un anion choisi dans le groupe constitué par la montmorillonite, la bentonite, l'hectorite, la kaolinite, la serpentine, le talc, la pyrophyllite, le mica, la phlogopite, la biotite, la muscovite, la paragonite, la vermiculite, la beidellite, la xantophyllite, la margarite, le feldspath, la zéolite, la wollastonite, l'actinolite, l'amosite, la crocidolite, la sillimanite, la nontronite, la smectite, la sépiolite, la saponite, la faujasite, la permutite et le sasil.

3. Utilisation selon la revendication 1, **caractérisée en ce que** l'ion ammonium est une des formules (a) à (j), 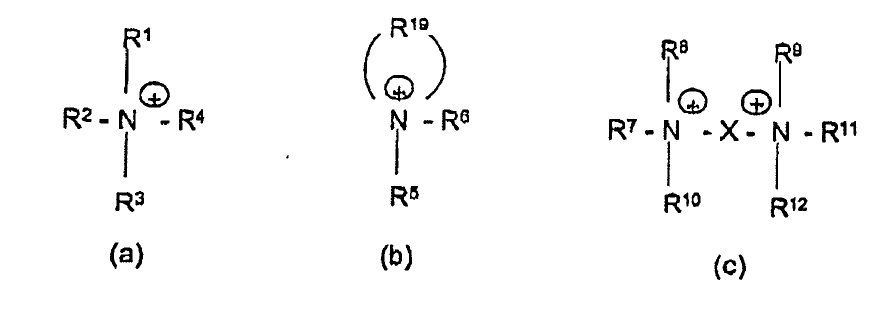 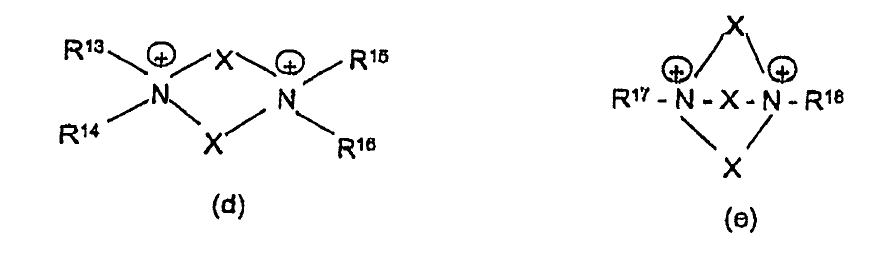 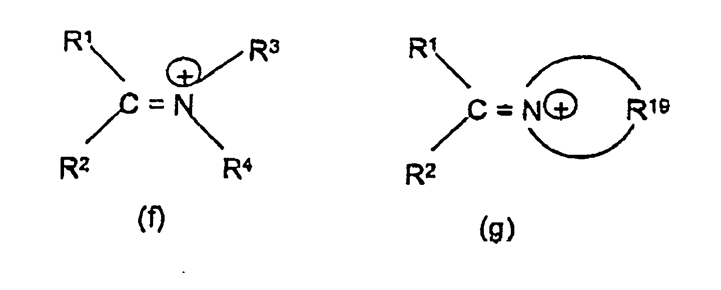 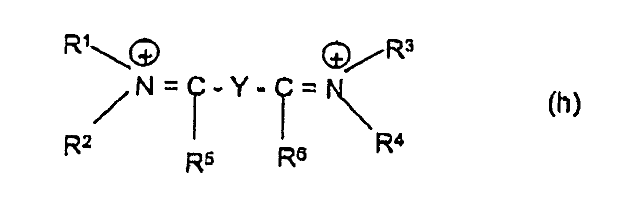 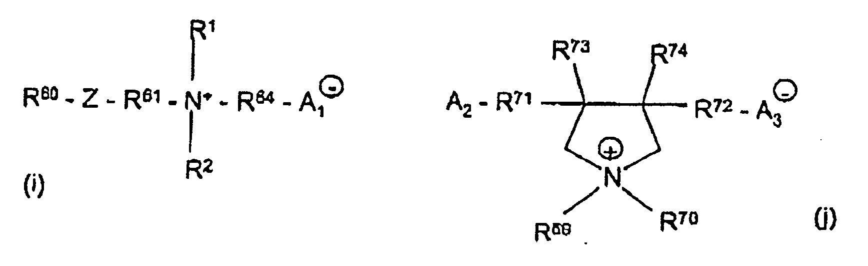 dans lesquels, R¹ à R¹⁸ sont identiques ou différents et désignent un hydrogène, CN, (CH₂)₁₋₁₈-CN, un halogène, un groupe alkyl en C₁-C₃₂ ramifié ou non ramifié, un ou plusieurs groupes alcényle en C₂-C₃₂ non saturés, un groupe alcoxy en C₁-C₂₂, un groupe hydroxyalkyle en C₁-C₂₂, un groupe halogénoalkyle en C₁-C₂₂, un groupe halogénoalkyle en C₂-C₂₂, un groupe aminoalkyle en C₁-C₂₂, un groupe trialkyle en (C₁-C₁₂)-ammonium-alkyle en (C₁-C₂₂);
un groupe alkylène en (C₁-C₂₂)-(C=O)O-alkyle en (C₁-C₃₂), un groupe alkylène en (C₁-C₂₂)-(C=O)O-aryle, un groupe alkylène en (C₁-C₂₂)-(C=O)NH-alkyle en (C₁-C₃₂), un groupe alkylène en (C₁-C₂₂)-(C=O)N-H-aryle, un groupe alkylène en (C₁-C₂₂)-O(CO)-alkyle en (C₁-C₃₂), un groupe alkylène en (C₁-C₂₂)-O(CO)-aryle; un groupe alkylène en (C₁-C₂₂)-NH(C=O)-alkyle en (C₁₋C₃₂), un groupe alkylène en (C₁-C₂₂)-NHCO-aryle;
où dans les liaisons esters acide ou amides acides 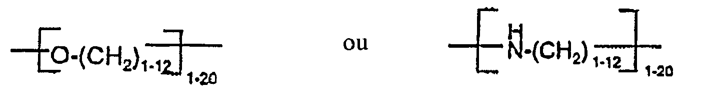 peuvent être intercalés ;
un groupe [alkylène-(C₁-C₁₂)-O-]₁₋₁₀₀-H ; un groupe aryle, un groupe alkylène en C₁-C₁₈-aryle ; un groupe -(O-SiR'₂)₁₋₃₂-O-SiR'₃, où R' a la signification d'un groupe alkyle en C₁₋₁₂, phényle, benzyle ou alcoxy en C₁-C₁₂ ; un groupe hétérocycle, un groupe hétérocyle-alkylène en C₁-C₁₈, où le reste aryle ou hétérocycle peuvent être substitués sur les carbones ou hétéroatomes par un ou plusieurs groupes alkyle en C₁-C₁₂, alcényle en C₁-C₄, alcoxy en C₁-C₄, hydroxy-alkyle en C₁-C₄, amino-alkyle en C₁-C₄, alkylamino en C₁-C₄, carboxy, hydroxy, amino, nitro, cyano, halogène, acyle en C₁-C₁₂, halogénoalkyle en C₁-C₄, alkylcarbonyle en C₁-C₄, alkylcarbonyloxy en C₁-C₄, alcoxycarbonyle en C₁-C₄, alkylaminocarbonyle en C₁-C₄, alkylcarbonylamino en C₁-C₄, arylcarbonyle C₆-C₁₀, aminocarbonyle, aminosulfonyle, alkylaminosulfonyle en C₁-C₄, phényle, naphtyle, héteroaryle;
R¹⁹ désigne un groupe alkylène en C₄-C₁₁, un groupe -(C₂H₄-O-)₁₋₁₇-(CH₂)₁₋₂-, un groupe -(C₂H₄-NR)₁₋₁₇-(CH₂)₁₋₂-, où R désigne l'hydrogène ou un alkyle en C₁-C₁₂; X a la signification de Y ainsi que de -CO-CH₂-CO-, 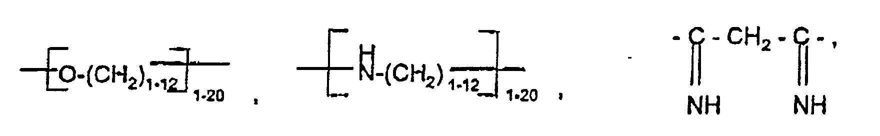 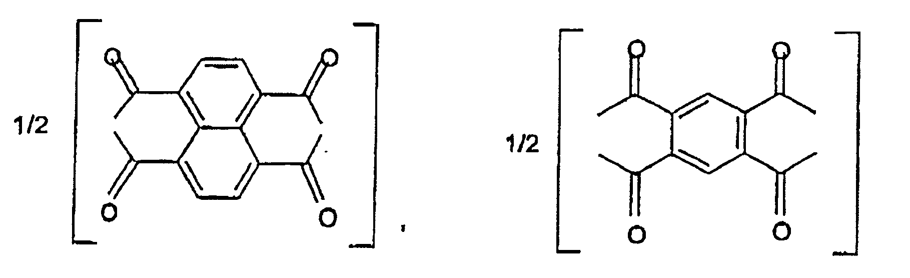 ou 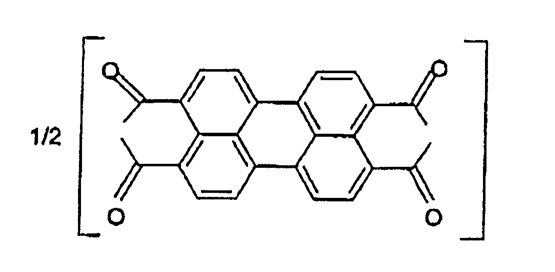 Y a la signification de 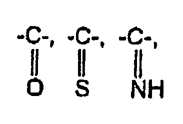 -(CH₂)₁₋₁₈-, 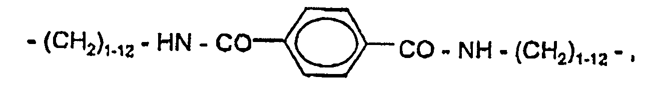 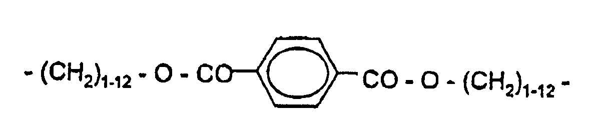 ou d'un aryle en C₆-C₁₄ o-, p-, m-, ou d'un hétéroaryle en C₄-C₁₄ avec 1, 2, 3 ou 4 hétéroatomes parmi le groupe N, O et/ou S.
R⁵⁰ représente un groupe acyle en C₁-C₃₂, alkyle en C₁-C₂₂, alkényle en C₂-C₂₂, alkylène en C₁-C₁₈-aryle en C₆-C₁₀, alkylène hétérocyle en C₁-C₂₂, aryle en C₆-C₁₀ ou hétéroaryle en C₄-C₁₄ avec 1, 2, 3 ou 4 hétéroatomes parmi le groupe N, O et/ou S,
R⁶¹ et R⁶⁴ représente un groupe -(CH₂)₁₋₁₈-, alkylène en C₁-C₁₂-arylène en C₆-C₁₀, arylène en C₆-C₁₀, alkylène-hétérocycle en C₀-C₁₂,
Z représente -NH- ou -O-,
A₁ et A₃ représentent -COO , -SO₃, -OSO₃, -SO₂, -COS ou -CS₂ ;
A₂ représente -SO₂Na, -SO₃Na, -SO₂H, -SO₃H ou l'hydrogène ;
R⁶⁹ et R⁷⁰ représentent, indépendamment l'un de l'autre, un hydrogène, un groupe alkyle en C₁-C₃₂, la chaîne alkyle pouvant contenir un ou plusieurs groupes -NH-CO-, -CO-NH-, -CO-O- ou -O-CO- ; alkylène en C₁-C₁₈-aryle, alkylène en C₀-C₁₈ hétérocyclique, hydroxyalkyle en C₁-C₁₈, halogénoalkyle en C₁-C₁₈, aryle, -(CH₂)₃-SO₃, 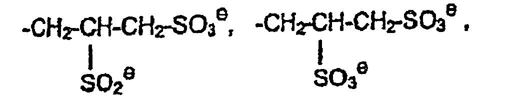 R⁷¹ et R⁷² représentent -(CH₂)₁₋₁₂- ;et
R⁷³ et R⁷⁴ représente un hydrogène ou un groupe alkyle en C₁-C₂₂.

4. Utilisation selon la revendication 3, **caractérisée en ce que** R¹ à R¹⁸ désignent un hydrogène, CN, CH₂-CN, CF₃, un groupe alkyle en C₁-C₂₂, un groupe alcényle en C₂-C₁₈, un groupe alcoxy en C₁-C₁₈, un groupe hydroxyalkyle en C₁-C₁₈, un groupe halogénoalkyle en C₁-C₁₈, un groupe halogénoalcényle en C₂-C₁₈, l'halogène désignant de préférence F ou Cl, un groupe aminoalkyle en C₁-C₁₈, un groupe trialkylammonium en C₁-C₆-alkyle en C₁-C₁₈, un groupe alkylène en C₁-C₁₈-O(C=O)-alkyle en C₁-C₂₂, un groupe alkylène en C₁-C₁₈-O(C=O)-phényle, un groupe alkylène en C₁-C₁₈-NHCO-alkyle en C₁-C₂₂, un groupe alkylène en C₁-C₁₈-NHCO-phényle, un groupe alkylène en C₁-C₁₈-(C=O)O-alkyle en C₁-C₂₂, un groupe alkylène en C₁-C₁₈-(C=O)O-phényle, un groupe alkylène en C₁-C₁₈-(C=O)NH-alkyle en C₁-C₂₂, un groupe alkylène en C₁-C₁₈-CONH-phényle, un groupe benzyle, un groupe phényle, un groupe naphtyle, un groupe alkylène en C₁-C₁₂ hétérocyclique ;
R¹⁹ désigne un groupe alkylène en C₄-C₅, -(C₂H₄-O)₁₋₉-(CH₂)₁₋₂-, -(C₂H₄-NH)₁-₉-(CH₂))₁₋₂ ;
R⁶⁰ désigne un groupe acyle en C₁-C₁₈, un groupe alkyle en C₁-C₁₈, un groupe alcényle en C₂-C₁₈, un groupe alkylène en C₁-C₁₂-phényle, un groupe alkylène en C₁-C₁₈-pyridyle, un groupe phényle, un groupe pyridyle ;
R⁶¹ et R⁶⁴ désignent -(CH₂)₁₋₁₂-, un groupe alkylène en C₁-C₈-phénylène, un groupe phénylène ; un groupe alkylènepyridylène en C₁-C₈ ou un groupe alkylpipéridylène ;
R⁷¹ et R⁷² désignent -(CH₂)₁₋₈ et
R⁷³ et R⁷⁴ désignent l'hydrogène ou un groupe alkyle en C₁-C₁₈.

5. Utilisation selon la revendication 1, **caractérisée en ce que** l'ion ammonium est un hétérocycle aliphatique ou aromatique à 5 à 12 chaînons ayant 1 à 4 atomes de N, O et/ou S dépendant du cycle, 2 à 8 cycles pouvant être annelés, de préférence les cycles pyridinium, pyridazinium, pyrimidinium, pyrazinium, purinium, tétraazaporphyrinium, pipéridinium, morpholinium, tétrazonium, triazacyclononanium et tétraazacyclododécanium.

6. Utilisation selon la revendication 1, **caractérisée en ce que** le complexe métallique cationique est un carboxylate métallique, un salicylate métallique, un sulfonate métallique, un complexe azo-métallique 1/1 ou un dithiocarbamate métallique, le métal étant de préférence Al, Mg, Ca, Sr, Ba, TiO, VO, Cr, V, Ti, Zr, Sc, Mn, Fe, Co, Ni, Cu, Zn et ZrO et le complexe métallique contenant le cas échéant un ou plusieurs ligands.

7. Utilisation selon une ou plusieurs des revendications 1 à 6, **caractérisée en ce que** le cation organique est un ion ammonium fluoré de formule (x) : 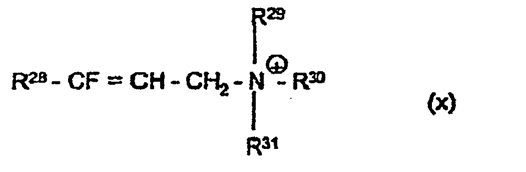 dans laquelle
R²⁸ désigne un groupe alkyle perfluoré ayant 5 à 11 atomes de carbone,
R²⁹, R³⁰ et R³¹ sont identiques ou différents et désignent un groupe alkyle ayant 1 à 5 atomes de carbone, de préférence 1 à 2 atomes de carbone.

8. Silicate structural de type sel, dans lequel le silicate est l'hectorite, la beidellite, l'illite, la muscovite, la xantophyllite, la margarite, la sépiolite, la saponite, le mica, le feldspath, la nontronite, la montmorillonite, la smectite, la bentonite, la faujasite, la zéolite A, X ou Y, la permutite, le sasil ou une combinaison de ceux-ci ; et le cation est un ion de formule (x) selon la revendication 7.

9. Procédé pour préparer un silicate structural de type sel selon la revendication 8, **caractérisé en ce qu'**on combine le silicate et le sel du cation de formule (x) dans un milieu aqueux.

10. Toner, poudre ou vernis en poudre électrophotographique, contenant 30 à 99,99 % en poids, de préférence 40 à 99,5 % en poids d'un liant, 0,01 à 50 % en poids, de préférence 0,05 à 20 % en poids d'au moins un silicate de type sel comme défini dans l'une des revendications 1 à 8 et le cas échéant, 0,001 à 50 % en poids, de préférence 0,05 à 20 % en poids d'un colorant, respectivement par rapport au poids total du toner, de la poudre ou du vernis en poudre électrophotographique.
